(19) Europäisches Patentamt / European Patent Office / Office européen des brevets

(11) **EP 3 345 598 A1**

(12) **EUROPEAN PATENT APPLICATION**

(43) Date of publication:
**11.07.2018  Bulletin 2018/28**

(21) Application number: **18150895.3**

(22) Date of filing: **09.01.2018**

(51) Int Cl.:
*A61K 31/407* (2006.01)   *A61P 29/00* (2006.01)
*A61K 9/00* (2006.01)   *A61K 9/08* (2006.01)
*A61M 5/142* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **09.01.2017   US 201762443853 P**

(71) Applicant: **Steadymed Ltd.**
**76701 Rehovot (IL)**

(72) Inventors:
• NOYMER, Peter
 **Los Gatos, CA California 95032 (US)**
• HURREY, Michael Laird
 **San Ramon, CA California 94583 (US)**

(74) Representative: **Fairbairn, Angus Chisholm**
**Marks & Clerk LLP**
**90 Long Acre**
**London WC2E 9RA (GB)**

(54) **DOSE SPARING KETOROLAC FORMULATIONS AND METHODS AND DEVICES FOR USE WITH SAME**

(57)    Provided is a unit dosage form of Ketorolac providing for a reduced dosage than typically provided to a subject in need of same, wherein said dosage form of Ketorolac is in a sterile fluid composition formulated for continuous subcutaneous delivery via body-worn infusion pump assembly, and wherein said Ketorolac is present at a concentration of between **40** mg/mL and **240** mg/mL formulated for single use delivery in a volume not to exceed 1-10 mL. Uses of same, body-worn infusion pump assemblies containing same and containers containing same for incorporation within a body-worn infusion pump assembly for a dose-sparing formulation comprising Ketorolac are also provided.

EP 3 345 598 A1

**Description**

*BACKGROUND OF THE INVENTION*

Ketorolac is a nonsteroidal anti-inflammatory drug and an inhibitor of prostaglandin synthesis, and thus possesses anti-inflammatory, analgesic and antipyretic activities.

The chemical name of Ketorolac is ($\pm$)-5-benzoyl-2,3-dihydro-1H-pyrrolizine-1-carboxylic acid, compound with 2-amino-2-(hydroxymethyl)-1,3-propanediol.

Pharmaceutical formulations with analgesic and antipyretic action, containing Ketorolac have been described, as has Ketorolac use for therapy in pain management, ocular pain treatment, periodontal affection treatments, and for the treatment of carcinomas of the scaly cells in the oral cavity or of the oropharynx.

Ketorolac formulations for oral, parenteral and topical administration have been described, as have several pharmaceutical forms, including tablets, suppository, pills, capsules, powder, solutions, suspensions, emulsions, creams, lotions and unguents.

Other formulations containing Ketorolac have been described, allowing for the application of Ketorolac through dentifrices, and including solutions for mouthwash and spray for oral cavity or dental solutions.

Nasal sprays containing Ketorolac have been described, as have transdermal formulations.

Formulations containing Ketorolac providing rapid anti-inflammatory, antipyretic and analgesic action include those via injection, yet the same are associated with illness and discomfort to the patient.

Given the importance in the use of Ketorolac as an analgesic, it is clear that compromising the speed of Ketorolac action is significantly undesirable, and the side effects associated with injectable forms of Ketorolac highlight the importance and need for providing a more efficacious and/or more tolerable Ketorolac treatment for patients.

*SUMMARY OF THE INVENTION*

It is an object of this invention to provide a more efficacious and/or more comfortable, consistent and reliable Ketorolac treatment for patients in need of same.

In some aspects of this invention, there is provided a method of providing analgesia, anti-pyretic effects or reducing pain in a subject presenting with a pain condition, comprising the steps of: administering Ketorolac in a sterile fluid composition at a dosage of between **40** mg/mL and **240** mg/mL to said subject and administering said Ketorolac to said subject via continuous subcutaneous delivery.

In some embodiments, the invention provides a method of reducing administration site irritation, inflammation or a combination thereof in a subject presenting with a pain condition, comprising the steps of: administering Ketorolac in a sterile fluid composition at a dosage of between 40 mg/mL and 240 mg/mL to said subject and administering said Ketorolac to said subject via continuous subcutaneous delivery

In some embodiments, the invention provides a method of providing analgesia, anti-pyretic effects, or reducing pain in a subject in need thereof, where said subject is administered Ketorolac in a sterile fluid composition in a continuous subcutaneous infusion at a rate of about 90 mg per 24 hours over period of 24 to 120 hours.

In some embodiments, the invention provides a method of providing analgesia, anti-pyretic effects, or reducing pain in a subject in need thereof, where said subject is administered Ketorolac in a sterile fluid composition at a dosage of about 30 mg subcutaneously in a period not exceeding 1 hour followed by a continuous subcutaneous infusion of 90 mg of ketorolac over the remaining 24 hours, followed by a continuous subcutaneous infusion of Ketorolac at a rate of 120 mg per 24 hours over an additional period of 24 to 96 hours.

In some embodiments, the invention provides a method of providing analgesia, anti-pyretic effects, or reducing pain in a subject in need thereof, where said subject is administered a bolus of Ketorolac in a sterile fluid composition at a dosage of about 30 mg intramuscularly followed by a continuous subcutaneous infusion of 90 mg of Ketorolac over the first 24 hours, followed by a continuous subcutaneous infusion of Ketorolac at a rate of 120 mg per 24 hours over a period of 24 to 96 hours.

According to these aspects, analgesia is achieved in a patient continuously during administration, and in some embodiments, effective analgesia is achieved with fewer side effects associated with use of other means of administering Ketorolac.

In some aspects, diminishing or abrogating certain side effects may in turn lead to greater analgesic effect, as well.

In some embodiments such methods result in the ability to achieve the desired analgesic, anti-inflammatory, anti-pyretic effect or combination thereof in the patient with a reduced dosage or sustained dosage over a significantly prolonged period of time, without at least some of the adverse effects previously reported with Ketorolac use.

In some embodiments, such method results in less of a need to administer higher Ketorolac dosages over time and in some embodiments, such method results in a less frequent need for adjustment of a dosage provided to such subject, over time, and in some embodiments, such method provides for a combination of these phenomena.

In some embodiments, such method results in an ability to administer Ketorolac for a longer duration than was previously achievable, without negative effect.

In some embodiments, such sustained dosage is attainable, due to reduced adverse effect, which in some aspects, may include reduced renal damage, reduced hepatic damage, and other side effects associated with the administration of sustained dosages in a given subject.

In some embodiments, such lowered or sustained dosage results in ultimate delivery of complete analgesia, which was not previously attainable in said subject. In some embodiments, such optimal dosage may be obtained faster and in a smaller volume in a subject, than was attainable with other Ketorolac compositions formulated for oral, nasal, or intravenous delivery, etc.

In some embodiments, adverse effects reduced or abrogated or prevented comprise gastrointestinal bleeding, inhibition of platelet function, renal impairment, or a combination thereof.

In some embodiments, the methods make use of a selectively activatable body-worn infusion -pump assembly comprising a sealed prefilled drug-reservoir containing a unit dosage form comprising Ketorolac to administer Ketorolac to subjects.

In some embodiments, the Ketorolac is formulated for single use delivery in a volume not to exceed 2-5 mL and in some embodiments, the Ketorolac is formulated for single use delivery in a volume not to exceed 3 mL. In some embodimetns, the Ketorolac is formulated for single use delivery in a volume not to exceed 1 mL.

In some embodiments, the Ketorolac is formulated in a sterile fluid composition that is ethanol free or sodium chloride free.

In some embodiments, the Ketorolac is administered Ketorolac over a period of time of more than 5 consecutive days.

This invention also provides a drug infusion pump loaded with an amount of Keterolac providing for a reduced dosage than typically provided, wherein said Keterolac is in a sterile fluid composition and said Ketorolac is present at a concentration of between 40 mg/mL and 240 mg/mL formulated for single use delivery in a volume not to exceed 1-10 mL, wherein the drug infusion pump is programmed to deliver the Keterolac via continuous subcutaneous delivery.

According to this aspect and in some embodiments, the drug infusion pump is a body-worn infusion pump and in some embodiments, the pump is a pre-filled and preprogrammed pump. In some embodiments, the drug infusion pump is preprogrammed to administer Ketorolac according a method as herein described.

It is to be understood that the term "patch pump" as referred to herein, is to be understood to relate to any or be exchangeable with any body-worn infusion pump.

In some embodiments, the pre-filled, pre-programmed pump is programmed to deliver Ketorolac at a dosage of between about 40 - 120 mg/day and in some embodiments, the Ketorolac is present at a concentration of between 120 mg/mL and 240 mg/mL. In some embodiments, the composition provides for a maximal volume of infusion which does not exceed 5 mL for single use. In some embodiments, the composition provides for a maximal volume of infusion which does not exceed 1 mL for single use.

In some embodiments, the said sterile fluid composition is ethanol-free.

In some embodiments, the composition comprises Disodium EDTA dihydrate as the preservative in said dosage form.

In some embodiments, the composition is at a pH of about from 7.6 to about 8.0.

In some embodiments, the Ketorolac is provided to said subject at a rate of less than 4 mL/day.

In some embodiments, the Ketorolac is Ketorolac tromethamine.

This invention also provides a unit dosage form of Ketorolac providing for a reduced dosage than typically provided, wherein said dosage form of Ketorolac is in a sterile fluid composition formulated for continuous subcutaneous delivery via pre-filled, pre-programmed pump assembly, and wherein said Ketorolac is present at a concentration of between 40 mg/mL and 240 mg/mL formulated for single use delivery in a volume not to exceed 1-10 mL, wherein the reduced dosage of Ketorolac provides a PK profile with Ketorolac blood levels between predetermined IM and PO dosing regimens based on simulation results from a MMSPK model at a dosage of less than 120 mg/day that provides effective analgesia, antipyretic effects or reduction of pain.

This invention also provides a unit dosage form of Ketorolac in a sterile fluid composition formulated for continuous subcutaneous delivery via pre-filled, pre-programmed pump assembly, wherein said Ketorolac is present at a concentration of between 40 mg/mL and 240 mg/mL formulated for single use delivery in a volume not to exceed 1-10 mL, wherein said composition is alcohol free, sodium chloride free, or a combination thereof, at a pH of about 7.5 - 8.5 and stable for a period of at least 12 months at room temperature.

According to these aspects and in some embodiments, the composition contains only a single preservative, which preservative is EDTA.

In some embodiments, a more concentrated fluid composition of Ketorolac is achieved by formulating the concentrated Ketorolac containing small volume sterile compositions as herein described, to be ethanol free and sodium chloride free and in some embodiments, such composition is significantly more stable over time than identical compositions containing ethanol and sodium chloride or compositions which are ethanol and sodium chloride free.

It is to be understood that reference to "Ketorolac" includes Ketorolac tromethamine and other pharmaceutically acceptable forms.

In some embodiments, the subject is treated with a lower dosage for a sustained period of time, or in some embodiments, the dosage is titrated downward over time.

In some aspects, this invention provides an aseptically filled, single-use container for delivery via pump the single-use container comprising a parenteral formulation of Ketorolac for continuous subcutaneous delivery to a subject in need of such treatment, which provides, in some embodiments, for an ability to deliver a loading dose of Ketorolac injected at the start of the infusion, whereby such bolus profile provides for efficacious blood levels quickly and in some embodiments, the same may promote or provide for a lower risk of side effects or reduced side effects.

In some embodiments, such reduced side effects include gastrointestinal bleeding, inhibition of platelet function, and renal impairment, and others, as will be appreciated by the skilled artisan.

In some embodiments, the combined device and formulation of this invention and methods of use of this invention provide for comparable efficacy obtained with other Ketorolac formulations, using lower Ketorolac dosages, as compared to other administration routes, or in some embodiments, for more consistent circulating levels of Ketorolac in a subject, or in some embodiments, for other advantages not achieved using any other known Ketorolac formulation.

In some embodiments, the combined device and formulation of this invention and methods of use of this invention provide for prolonged, relatively consistent delivery of Ketorolac to a subject in need, which in turn, in embodied aspects, provides for a more optimal therapeutic profile, including better analgesia to the subject.

In some aspects of this invention, the formulation is further adapted to contain specific stabilizers in an ethanol free or sodium chloride free setting, which provides, in some embodiments, for enhanced formulation stability, and in some embodiments, such enhanced stability is seen even when the formulation is stored at room temperature and in some aspects, such stability is seen even when the formulation is stored at higher temperatures, such as, for example, that experienced in the United States in various states, in various seasons outdoors, etc..

In some aspects, this invention provides a formulation of Ketorolac for subcutaneous administration or for continuous subcutaneous administration, which formulation has a maximal volume of infusion which does not exceed 10 mL. In some aspects, the delivery volume is 1 mL per day for a normal adult and in some embodiments, the delivery volume is from 0.5-5 mL per day. In some embodiments the delivery volume is from 1-3 mL per day.

In some embodiments, this invention provides a sterile fluid composition for continuous subcutaneous delivery via infusion pump assembly, said composition comprising Ketorolac at a concentration of between **40** mg/mL and **240** mg/mL in a volume not to exceed 2-5 mL.

In some embodiments, this invention provides a unit dosage form of Ketorolac in a sterile fluid composition formulated for continuous subcutaneous delivery via infusion pump assembly, wherein said Ketorolac is present at a concentration of between **40** mg/mL and **240** mg/mL formulated for single use delivery in a volume not to exceed 2-5 mL.

In some aspects, this invention provides a formulation of Ketorolac for continuous subcutaneous administration, which formulation delivers 240 mg/ml.

In some aspects, this invention provides a formulation of Ketorolac for continuous subcutaneous administration, which formulation delivers 120 mg/ml.

In some aspects, this invention provides a formulation of Ketorolac for continuous subcutaneous administration, which formulation delivers 90 mg/ml.

In some aspects, this invention provides a formulation of Ketorolac for continuous subcutaneous administration, which formulation delivers 60 mg/ml.

In some aspects, the Ketorolac formulation provides for a dose-sparing effect. In some aspects, the adult daily dosage may be provided as from 60 - 120 mg/day. In some aspects, the adult daily dosage may be provided as from 60 - 90 mg/day.

In some aspects, the adult daily dosage typically used for normal weight, non-elderly patients is 120 mg/day, and a daily dosage 20% - 40% less than same can be provided with the formulations and delivery system of this invention. For example, in some embodiments, formulations providing a daily dosage of 80-90 mg/day are envisioned. In some aspects, the daily dosage for elderly, or underweight patients typically used is 60 mg/day, which as well may be reduced by from 20% - 40% less, by using the formulations and delivery systems of this invention. For example, in some embodiments, formulations providing a daily dosage of 40-45 mg/day are envisioned.

In some aspects, the invention provides for a single use container comprising the described Ketorolac formulation for delivery by infusion pump, which formulation provides for fewer side effects experienced by the patient in use of same.

In some aspects, the invention provides for a single use container comprising the described Ketorolac formulation for delivery by infusion pump, which formulation provides for greater formulation stability and/or less oxidation as compared to other formulations of Ketorolac in use to date.

*BRIEF DESCRIPTOIN OF THE DRAWINGS*

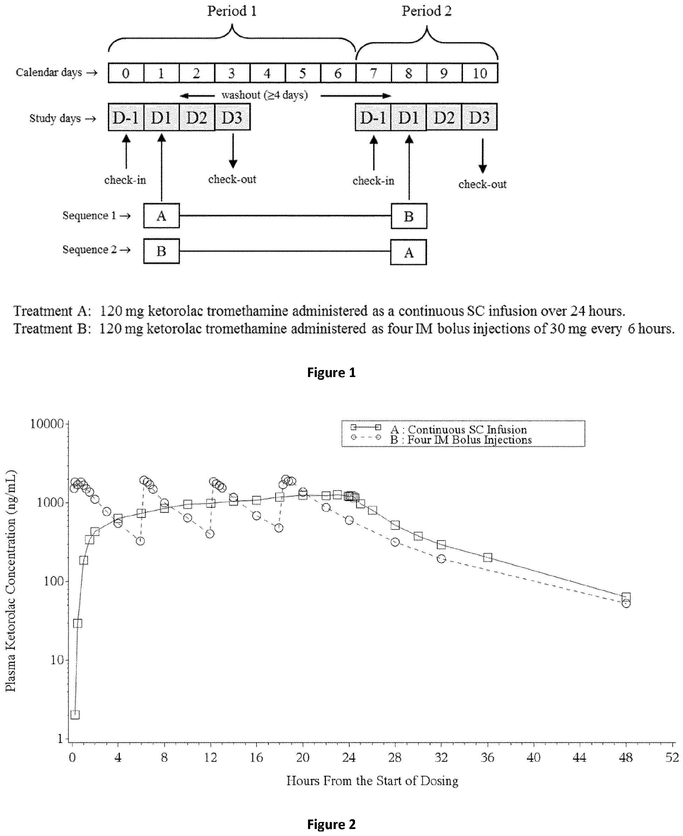

Figure 1 schematically depicts the study design scheme for continuous subcutaneous Ketorolac infusion versus bolus intramuscular injection. Treatment A consisted of 120 mg Ketorolac tromethamine administered as a continuous SC infusion over 24 hours, while Treatment B consisted of 120 mg Ketorolac tromethamine administered in four bolus intramuscular injections of 30 mg every 6 hours.

Figure 2 plots the mean plasma Ketorolac concentration over time profiles as a function of the treatment regimen conducted. The scale provided is a semi-log scale, with treatment A being administered to 11 patients and treatment B being administered to 12 patients.

*DETAILED DESCRIPTION OF THE INVENTION*

This invention aims to provide a Ketorolac treatment for patients in need of same, which is delivered over time via continuous subcutaneous administration, which Ketorolac is formulated for continuous subcutaneous delivery in a sterile, single use fluid composition, providing, inter alia, analgesia, an antipyretic or anti-inflammatory effect to said subject, and in some embodiments, providing for reduced adverse effects than seen to date in subjects receiving alternate formulations of Ketorolac.

In some embodiments, the Ketorolac is formulated for delivery of a lower dosage than alternate formulations of Ketorolac, or in some embodiments, the subject is treated with a lower dosage for a sustained period of time than is typically administered in patients receiving Ketorolac therapy, or in some embodiments, the dosage is titrated downward over time, which in some embodiments, is due to reduced adverse effects than what is typically experienced with other Ketorolac treatment regimens in affected subjects.

In some embodiments, Ketorolac is provided in a single use container that provides therapy for one or several days at a time, and where such container can supply Ketorolac at a rate of 40-240 mg/ml, in a delivery volume of 1-5 mL per day. In some embodiments, the Ketorolac is provided as two or more single use containers that provide such therapy.

Surprisingly, providing Ketorolac by continuous subcutaneous infusion is associated not only with optimal analgesia, anti-pyretic effects and reduced severe irritation/inflammation in the subject, but the same may be accomplished using lower drug dosages, and being accompanied by fewer adverse effects. Furthermore, surprisingly, the same is further associated with an improved responsiveness to Ketorolac. In some aspects, such improved performance is seen in the ability to provide sustained use of a relatively low dose, or in some embodiments, a slower time to increasing such dosage, or in some aspects, a titration downward of a dosage, or in some embodiments, a less frequent need for administration of Ketorolac, or in some embodiments, any combination of these effects.

In some aspects, the Ketorolac formulation provides for a dose-sparing effect. In some aspects, the adult daily dosage may be provided as from 40 - 240 mg/day. In some aspects, the adult daily dosage typically used for normal weight, non-elderly patients is 120 mg/day, and a daily dosage 25% - 35% less than same can be provided with the formulations and delivery system of this invention. For example, in some embodiments, formulations providing a daily dosage of 80-90 mg/day are envisioned. In some aspects, the daily dosage for elderly, or underweight patients typically used is 60 mg/day, which as well may be reduced by from 25% - 35% less, by using the formulations and delivery systems of this invention. For example, in some embodiments, formulations providing a daily dosage of 40-45 mg/day are envisioned.

In some embodiments, a similar dose sparing effect is achievable in pediatric formulations, and the daily dosage will be titrated downward, accordingly, as will be appreciated by the skilled artisan, with the same ability to reduce such pediatric daily dosage by from 10-35% less than that typically in use via other administration routes/formulation protocols.

Surprisingly, providing Ketorolac by continuous subcutaneous injection/infusion as described herein provided for a product with greater long-term stability/shelf life as typically seen in other formulations for parenteral delivery.

Therefore, this invention provides, *inter alia,* a method of providing analgesia, anti-pyretic effects or reducing pain in a subject presenting with a pain condition, comprising the steps of: administering Ketorolac in a sterile fluid composition at a dosage of between **40** mg/mL and **240** mg/mL to said subject and administering said Ketorolac to said subject via continuous subcutaneous delivery. This invention provides, in other embodiments, a method of reducing administration site irritation, inflammation or a combination thereof in a subject presenting with a pain condition, comprising the steps of: administering Ketorolac in a sterile fluid composition at a dosage of between 40 mg/mL and 240 mg/mL to said subject and administering said Ketorolac to said subject via continuous subcutaneous delivery.

In still further embodiments, the invention provides a method of providing analgesia, anti-pyretic effects, or reducing pain in a subject in need thereof, where said subject is administered Ketorolac in a sterile fluid composition in a continuous subcutaneous infusion at a rate of about 90 mg per 24 hours over period of about 24 to 120 hour.

In some embodiments, the invention provides a method of providing analgesia, anti-pyretic effects, or reducing pain in a subject in need thereof, where said subject is administered Ketorolac in a sterile fluid composition at a dosage of about 30 mg subcutaneously in a period not exceeding 1 hour followed by a continuous subcutaneous infusion of 90 mg of Ketorolac over the remaining 24 hours, followed by a continuous subcutaneous infusion of Ketorolac at a rate of 120 mg per 24 hours over an additional period of 24 to 96 hours.

In some embodiments, the invention provides a method of providing analgesia, anti-pyretic effects, or reducing pain in a subject in need thereof, where said subject is administered Ketorolac in a sterile fluid composition in an intravenous bolus at a dosage of about 30 mg followed by a continuous subcutaneous infusion of 90 mg of Ketorolac about or over 24 hours, followed by a continuous subcutaneous infusion of Ketorolac at a rate of 120 mg per 24 hours over an additional period of about 24 to 96 hours

In some embodiments, the invention provides a method of providing analgesia, anti-pyretic effects, or reducing pain in a subject in need thereof, where said subject is administered a bolus of Ketorolac in a sterile fluid composition at a dosage of about 30 mg intramuscularly followed by a continuous subcutaneous infusion of 90 mg of Ketorolac over the first 24 hours, followed by a continuous subcutaneous infusion of Ketorolac at a rate of 120 mg per 24 hours over a period of 24 to 96 hours.

In some aspects, the bolus administration followed by continuous subcutaneous infusion unexpectedly provides not only more rapid analgesia, but sustained analgesia, which in some aspects is provided at a lower dosage than previously attainable.

The analgesia achieved in a patient via the methods/compositions/devices as herein described, is attained continuously during administration, and in some embodiments, effective analgesia is achieved with fewer side effects associated with use of other means of administering Ketorolac.

In some aspects, diminishing or abrogating certain side effects may in turn lead to greater analgesic effect, as well.

In some embodiments such methods result in the ability to achieve the desired analgesic, anti-inflammatory, anti-pyretic effect or combination thereof in the patient with a reduced dosage or sustained dosage over a significantly prolonged period of time, without at least some of the adverse effects previously reported with Ketorolac use.

In some embodiments, such method results in less of a need to administer higher Ketorolac dosages over time and in some embodiments, such method results in a less frequent need for adjustment of a dosage provided to such subject, over time, and in some embodiments, such method provides for a combination of these phenomena.

In some embodiments, such method results in an ability to administer Ketorolac for a longer duration than was previously achievable, without negative effect.

In some embodiments, such sustained dosage is attainable, due to reduced adverse effect, which in some aspects, may include reduced renal damage, reduced hepatic damage, and other side effects associated with the administration of sustained dosages in a given subject.

In some embodiments, such lowered or sustained dosage results in ultimate delivery of complete analgesia, which was not previously attainable in said subject. In some embodiments, such optimal dosage may be obtained faster and in a smaller volume in a subject, than was attainable with other Ketorolac compositions formulated for oral, nasal, or intravenous delivery, etc..

In some embodiments, adverse effects reduced or abrogated or prevented comprise gastrointestinal bleeding, inhibition of platelet function, renal impairment, or a combination thereof.

In some embodiments, the methods make use of a selectively activatable body-worn infusion -pump assembly comprising a sealed prefilled drug-reservoir containing a unit dosage form comprising Ketorolac to administer Ketorolac to subjects.

In some aspects of the invention, there is provided a unit dosage form of Ketorolac comprising an aseptically filled drug reservoir that may be stored unopened at room temperature for at least 18 months, or in some embodiments, for at least 24 months, or in some embodiments for at least 7 days, or in some embodiments for up to 12 months. In some aspects, such enhanced formulation stability provides an additional advantage to the instant formulation, in addition to other advantages as herein described.

In some aspects of the invention, there is provided a unit dosage form of Ketorolac comprising an aseptically filled drug reservoir suitable for continuous infusion for a period for more than 24 hours. In some embodiments, there is provided a unit dosage form of Ketorolac comprising an aseptically filled drug reservoir suitable for continuous infusion for a period of from 24 hours to 48 hours, or from 36 hours to 72 hours, or from about 24 hours to about 5 days.

In some aspects of the invention, there is provided a unit dosage form of Ketorolac comprising an aseptically filled drug reservoir suitable for continuous infusion, where the Ketorolac formulation comprises 0.5-5 mg/mL, or in some embodiments, 1-3 mg/mL, or in some embodiments, about 2-3 mg/mL, or in some embodiments, about 2 mg/mL sodium EDTA, which in some embodiments is further characterized as being sodium chloride free, or in some embodiments, ethanol free, or in some embodiments, characterized by a pH of greater than 7.5, or in some embodiments, having a pH from about 7.5 - 8.5, or in some embodiments, having a pH from about 7.5 - 8.3, or in some embodiments, having a pH from about 7.8 - 8.3.

In some aspects of the invention, there is provided a unit dosage form of Ketorolac comprising an aseptically filled drug reservoir suitable for continuous infusion, where the Ketorolac formulation is characterized by a tonicity of between 160 and 270 mOsmol/kg for a formulation comprising 45 mg/mL Ketorolac and in some embodiments, the tonicity is between 360 and 500 mOsmol/kg for a formulation comprising 90 mg/mL Ketorolac.

In some aspects of the invention, there is provided a unit dosage form of Ketorolac comprising an aseptically filled drug reservoir suitable for continuous infusion, where the aseptically filled drug reservoir is characterized by a diameter at least 3 times its height, or in some embodiments, from at least 3 times to about 50 times its height, and in some embodiments, having a volume of 1 mL to 3 mL or in some embodiments, having a volume of from about 1 mL to 5 mL.

In some aspects of the invention, there is provided a unit dosage form of Ketorolac comprising an aseptically filled drug reservoir suitable for continuous infusion, where the surfaces of said reservoir walls comprises a plastic from the group including COP (cyclic olefin polymer) and COC (cyclic olefin copolymer).

In some aspects, there is provided a unit dosage form of Ketorolac comprising an aseptically filled drug reservoir where no more than 3% of the Ketorolac contained therein degrades over a period of 3 months when stored at 25 degrees and 60% humidity and in some embodiments, no more than 3% of the Ketorolac contained therein degrades over a period of 3 months when stored at 40 degrees and 75% humidity.

In some aspects of this invention, there is provided an aseptically filled, single-use container for delivery via pump the single-use container comprising a parenteral formulation of Ketorolac for continuous subcutaneous delivery to a subject in need of such treatment, which provides, in some embodiments, for an ability to deliver a loading dose of Ketorolac injected at the start of the infusion, whereby such bolus profile provides for efficacious blood levels quickly while also incurring a lower risk of side effects or reduced side effects.

The Ketorolac as used in the compositions, devices and methods of the present invention are conveniently prepared by methods the same as or analogous to those described in U.S. Pat. No. United States Patent 4,089,969, 5,532,38; 6,191,285; 6,197,976; 6,323,344; each of which is hereby incorporated by reference in its entirety, or any other appropriate method, as will be appreciated by the skilled artisan.

The present invention extends to compositions, devices and methods of using physiologically acceptable salts of Ketorolac, as well as non-physiologically acceptable salts of Ketorolac that may be used in the preparation of the pharmacologically active compositions of the invention.

The term "pharmaceutically acceptable salt" refers to a salt of Ketorolac with an inorganic base, organic base, inorganic acid, organic acid, or basic or acidic amino acid. Salts of inorganic bases can be, for example, salts of alkali metals such as sodium or potassium; alkaline earth metals such as calcium and magnesium or aluminum; and ammonia. Salts of organic bases can be, for example, salts trimethylamine, triethylamine, pyridine, picoline, ethanolamine, diethanolamine, tromethamine, and triethanolamine. Salts of inorganic acids can be, for example, salts of hydrochloric acid, hydroboric acid, nitric acid, sulfuric acid, and phosphoric acid. Salts of organic acids can be, for example, salts of formic acid, acetic acid, trifluoroacetic acid, fumaric acid, oxalic acid, lactic acid, tartaric acid, maleic acid, citric acid, succinic acid, malic acid, methanesulfonic acid, benzenesulfonic acid, and p-toluenesulfonic acid. Salts of basic amino acids can be, for example, salts of arginine, lysine and ornithine. Salts of acidic amino acids can include, for example, salts of aspartic acid and glutamic acid. Quaternary ammonium salts can be formed, for example, by reaction with lower alkyl halides, such as methyl, ethyl, propyl, and butyl chlorides, bromides, and iodides, with dialkyl sulphates, with long chain halides, such as decyl, lauryl, myristyl, and stearyl chlorides, bromides, and iodides, and with aralkyl halides, such as benzyl and phenethyl bromides.

The present invention specifically envisions devices and methods of using same for the continuous subcutaneous delivery to a subject of a Ketorolac-containing formulation as herein described, including, in some embodiments, providing for a bolus administration of same followed by such continuous subcutaneous delivery. In some aspects, the devices envisioned will comprise some or all of the elements as described in U.S. Patent Number 8,834,454, U.S. Patent Application Publication Number 2009-0093772, U.S. Patent Application Publication Number 2014/0148761, each of which is fully incorporated herein by reference.

In some embodiments, reference to the compositions, kits and methods/uses of the invention, which refer to the term "comprise" or "comprising" also encompasses "consist of" or "consisting of".

In some embodiments, the unit dosage forms, compositions, kits and methods consist essentially of Ketorolac, in a formulation, whereby the term "consist essentially of" specifically excludes an additional active ingredient with antipyretic, or anti-inflammatory activity. In some embodiments, the term "consist essentially of" specifically excludes an additional active ingredient with anti-oxidant activity.

In some embodiments, the unit dosage forms, compositions, kits and methods comprise or make use of Ketorolac. In some embodiments, the dosage forms, compositions, kits and methods consist or make use of Ketorolac. In some embodiments, the dosage forms, compositions, kits and methods consist essentially of or make use essentially of Ketorolac.

In some embodiments, the term "comprise" refers to the inclusion of the indicated active agent, such as Ketorolac, as well as inclusion of other active agents, and pharmaceutically acceptable carriers, excipients, emollients, antioxidants, stabilizers, etc., as are known in the pharmaceutical industry.

In some embodiments, the compositions of this invention will consist essentially of an active Ketorolac ingredient. In some embodiments, the term "consisting essentially of" refers to a composition whose only active ingredient of a particular class of agents, is the indicated active ingredient, i.e. the only active NSAID is Ketorolac, however, other compounds may be included which are involved directly in the therapeutic effect of the indicated active ingredient. In some embodiments, with reference to the compositions of this invention, when referring to a composition consisting essentially of an active Ketorolac ingredient, such reference specifically excludes the incorporation of any other NSAID in the composition.

"Pharmaceutical composition" or "composition" means a composition containing one or more drugs or prodrugs, and optionally one or more excipients, as well as any product which results, directly or indirectly, from combination, complexation or aggregation of any two or more of the excipients and/or the drug or prodrug, or from dissociation of one or more of the excipients and/or drug and/or prodrug, or from other types of reactions or interactions of one or more of the excipients and/or drug and/or prodrug. Accordingly, the pharmaceutical composition of the present invention encompasses any composition obtainable by admixing a carrier-linked Ketorolac of the present invention and a pharmaceutically acceptable excipient.

The term "excipient" refers to a diluent, adjuvant, or vehicle with which the carrier-linked Ketorolac is administered. Such pharmaceutical excipient can be sterile liquids, such as water and oils. In some aspects, saline and aqueous dextrose are preferred excipients when the pharmaceutical composition is administered intravenously.

Saline solutions and aqueous dextrose and glycerol solutions are preferably employed as liquid excipients for injectable/infusion solutions.

The composition, if desired, can also contain minor amounts of wetting or emulsifying agents, pH buffering agents, like, for example, acetate, succinate, tris, carbonate, phosphate, HEPES (4-(2- hydroxyethyl)-1-piperazineethanesulfonic acid), MES (2-(N-morpholino)ethanesulfonic acid), or can contain detergents, like Tween, poloxamers, poloxamines, CHAPS, Igepal, or amino acids like, for example, glycine, lysine, or histidine.

Examples of suitable pharmaceutical excipients are described in "Remington's Pharmaceutical Sciences" by E.W. Martin. Such compositions will contain a therapeutically effective amount of Ketorolac in the form of at least one carrier- linked Ketorolac prodrug of the present invention, preferably in purified form, together with a suitable amount of excipient so as to provide the form for proper administration to the patient. The formulation should suit the mode of administration.

The term "pharmaceutically acceptable" means approved by a regulatory agency such as the EMA (Europe) and/or the FDA (US) and/or any other national or regional regulatory agency for use in animals, preferably in humans.

In some aspects, the composition will comprise an anti-oxidant. In some embodiments, the anti-oxidant is selected for instance from acids and theirs salts, vitamins and derivatives, amino acids, sulfites or free phenolic radical scavengers. When the antioxidant is an acid or a salt thereof, it may be selected for instance from ascorbic acid or its salts such as sodium ascorbate, isoascorbic acid or its salts such as sodium isoascorbate, citric acid or its salts such as sodium citrate, lactic acid or malic acid.

When the antioxidant is a vitamin or a vitamin derivative, it may be selected for instance from tocopherol (vitamin E), riboflavin (vitamin B2), tocopherol-PEG-succinate (vitamin derivative) or trolox (vitamin derivative).

When the antioxidant is an amino acid, it may be selected for instance from cysteine, tryptophane, histidine, selenocysteine, N-acetyl cysteine, taurine, glutathione or glutathione-glutathione.

When the antioxidant is a sulfite, it may be selected for instance from sodium sulfite or sodium metabisulfite.

When the antioxidant is a phenolic free radical scavenger, it may be selected for instance from butylated hydroxy toluene, butyl hydroxyl anisole or cinnamic acid.

In some aspects, the amount of Ketorolac or its derivative, or a pharmaceutically acceptable salt thereof, which is provided in a medication or kit according to the invention is sufficient to achieve the desired effect, which in some aspects, will depend on the concentration of the compound used, and the weight and condition of the patient.

For example, and providing guidance for the envisioned formulations and kits and applications in the methods of this invention, a daily dose may be in the range supplying Ketorolac at a rate of about 80 - 90 mg per day in normal adults, or a daily dose may be in the range supplying Ketorolac at a rate of about 40 - 50 mg per day in elderly or underweight adults, or those with renal impairment.

For example, a subcutaneous dose may be provided in a unit dosage form administered continuously, supplying a daily dosage of about 80 - 90 mg in 1 mL, or 40-50 mg in 1mL, or 120-240 mg in 2-5mL, which may conveniently be administered as an infusion, for example, using a pump compatible with the single-use container. For example, a subcutaneous dose may be provided in a unit dosage form administered continuously, supplying a daily dosage of about 80 - 90 mg in 0.5 - 3 mL, or 40-50 mg in 0.5 - 5 mL, or 120-240 mg in 0.5 - 5 mL, which may conveniently be administered as an infusion, for example, using a pump compatible with the single-use container.

In some aspects, a subcutaneous dose may be provided in a unit dosage form supplying a daily dosage of from 40-240 mg, which dosage may still further be increased as needed, or in some embodiments, decreased as needed. In some embodiments changes in dosage may be implemented over a much prolonged period without ill effect, or in some embodiments, a lower dosage is necessary to achieve the desired therapeutic effect.

In some aspects, infusion fluids suitable for this purpose contain, for example, a delivery dosage of from 40-240 mg per day, provided in a minimal volume, which does not exceed 10 mL, and in some aspects is in a volume that is about 0.5 - 5 mL, and in some aspects is in a volume that is about 1-3 mL, or less.

In the manufacture of a medicament according to the invention, hereinafter referred to as a "formulation," Ketorolac and/or its derivatives, and/or pharmaceutically acceptable salts thereof, may be admixed with, inter alia, an acceptable carrier. The carrier must, of course, be acceptable in the sense of being compatible with any other ingredients in the formulation and must not be deleterious to the subject. One or more of Ketorolac or its derivatives, or pharmaceutically acceptable salts thereof, may be incorporated in the formulations of the invention, which may be prepared by any of the well-known techniques of pharmacy for admixing the components.

As will be appreciated by the skilled artisan, the terms "subject" and "patient" are used interchangeable and refer to any subject in need of or benefitting in any way from the described treatment/administration protocol/method of kit provided by the instant invention.

This invention provides, in some aspects, a selectively activatable body-worn infusion -pump assembly comprising a sealed prefilled drug-reservoir containing the unit dosage form of Ketorolac in a sterile fluid composition formulated for subcutaneous infusion as herein described.

In some aspects, this invention provides an automatic infusion device comprising a sealed prefilled drug-reservoir containing the unit dosage form of Ketorolac in a sterile fluid composition, formulated for continuous subcutaneous infusion as herein described.

In some embodiments, the term "automatic infusion device" refers to a device that enables an individual (also referred to herein as a user or a patient or subject) to self-administer a dosage of a substance, such as a liquid medication, wherein the device differs from a standard syringe by the inclusion of a mechanism for automatically delivering the medication to the individual by infusion when the mechanism is engaged.

In some aspects, as will be appreciated by the skilled artisan, any appropriate automatic infusion device may be used, for example, as described in U.S. Pat. Nos. 3,910,260; 4,004,577; 4,689,042; 4,755,169; 4,795,433; 3,941,130; 4,261,358; 5,085,642; 5,092,843; 5,102,393; 5,267,963; 6,149,626; 6,270,479; 8,679,061 and 6,371,939, each of which is incorporated by reference herein in its entirety.

In some embodiments, PK-PD modeling suggests that blood level of 5 mcg/mL represents an approximate safety threshold for the drug. With the available intermittent/bolus dosing regimen for Ketorolac, high spikes in blood levels have been reported. Surprisingly, using the device/formulation of Ketorolac and via the methods described herein, blood level fluctuations, in some embodiments are reduced or abrogated, and in other embodiments, exceeding a 5 mcg/mL threshold is without significant deleterious effect. In some embodiments, per the PK-PD analysis described further herein below, the use of an infusion Pump for the delivery of Ketorolac improves the side-effect profile of therapies with this drug by e.g. reducing the incidence or severity of the side-effects noted with such use.

In some embodiments, the invention provides Ketorolac formulations and formulated for use with infusion devices as herein described, which provide greater shelf-life in terms of their long-term stability.

In some embodiments, the invention provides Ketorolac formulations and formulated for use with infusion devices as herein described, which are much more concentrated in terms of the Ketorolac concentration as compared to Ketorolac formulations comprising the state of the art. Surprisingly, such highly concentrated compositions are stable and provide for greater long-term storage than other existing Ketorolac formulations.

In some embodiments, the invention provides Ketorolac formulations and formulated for use with infusion devices as herein described, which include use of same in a pre-programmed pump containing device as described, such that a loading dose and the basal dose are provided to a subject, with the controls for same provided in a single unit. In some embodiments, such arrangement provides for controlled delivery without human intervention required to shift from bolus to basal level delivery, providing for the bolus delivery to quickly reach efficacious levels, without increasing the risk of side effects in the subject thus treated.

In some aspects, the invention provides Ketorolac formulations and formulated for use with infusion devices as herein described, which increase the local tolerance of the subject to Ketorolac, which may provide additional advantages, such as, for example, treatment, the avoiding in situ precipitation when formulating more concentrated compositions; increasing the chemical stability of the formulation and providing for an increased drug concentration allowing the reduction of the infusion volume.

In some aspects, this invention provides a kit comprising a formulation of Ketorolac for continuous subcutaneous delivery to a subject in need of such treatment, which provides analgesic effects, antipyretic effects or anti-inflammatory effects. In some aspects, such kits may provide multiple sterile formulations of Ketorolac for continuous subcutaneous delivery, which vary in terms of the dosage delivered of same.

In some aspects, such kits comprise a container, which may include a vial, for infusion, or in some embodiments, one or more prefilled drug-reservoirs containing the Ketorolac formulation comprising Ketorolac in a pharmaceutically acceptable carrier. In some aspects, the kits may further comprise instructions, such as a product insert or label, directing the user regarding proper administration and use of the formulation, or in some embodiments, instructions for making use of the formulations varying, for example in terms of Ketorolac dosage provided with said kit, which in some embodiments, provides instructions for titrating a dosage of same.

In some aspects, such kits may comprise a tubing set to connect the drug reservoir to the patient's cannula or catheter (also referred to herein as the means of administration), or in some embodiments, the means of administration is provided to the patient integrated with the container.

In some embodiments, the invention provides a method of reducing pain or providing analgesia in a subject in need thereof, wherein said subject is administered the unit dosage form of Ketorolac in a sterile fluid composition as herein described or in some embodiments, the subject is administered Ketorolac by making use of a kit as herein described.

In some embodiments, the invention provides a method of reducing irritation, inflammation or a combination thereof in a subject in need thereof, wherein said subject is administered the unit dosage form of Ketorolac in a sterile fluid composition as herein described or in some embodiments, the subject is administered Ketorolac by making use of a kit as herein described.

In some embodiments, the present invention overcomes previous restricted use of Ketorolac in terms of dosages and duration of therapy allowed in treated subjects, providing sustained delivery via the formulations as described herein and via use of the selectively activatable body-worn infusion -pump assemblies comprising Ketorolac as described herein.

In some aspects the present invention overcomes previous limitations in terms of the existing dilute Ketorolac formulations and the inclusion of ethanol in same, by providing the ability for more concentrated, low ethanol or ethanol free formulations.

In some embodiments, such advantages, in turn, may provide for Ketorolac-containing compositions with greater stability, or in some embodiments, such advantages may comprise the ability to deliver a unit dosage container which is smaller, and provide for greater ease and flexibility with respect to delivery systems/devices containing same.

In some embodiments, the present invention overcomes previous limitations in terms of the existing Ketorolac formulations and the inclusion of ethanol in same, by obviating or reducing side effects, such as irritation and inflammation, or administration site pain as a result of or as contributed to by the inclusion of ethanol in such formulations.

In some embodiments, the present invention overcomes previous limitations in terms of the existing Ketorolac formulations, in providing analgesia over a more extended period of time. In some embodiments, treatment beyond 5 consecutive days is uniquely achievable using the embodied dosage forms, compositions and body-worn infusion pump assemblies containing same. In some aspects, the specific drug delivery assemblies envisioned for use with the embodied dosage forms and compositions as herein described allow for slim profile more convenient devices, in part due to the ability to prepare more concentrated stable Ketorolac containing compositions.

In some aspects, the present invention thus provides a concentrated, stable and low-ethanol or ethanol free formulation, which in some embodiments, is provided as part of a compact subcutaneous delivery system, allowing for safer and more tolerated Ketorolac delivery, with for example, safer dosing and delivery sustained over longer periods of time than currently possible.

In some aspects, the present invention promotes the ability to arrive at more concentrated Ketorolac containing formulations, and in some aspects, the present invention provides for highly precise, controlled delivery systems to ensure safe dosing of such concentrated formulations, promoting optimal Ketorolac delivery.

In some aspects, the Ketorolac-containing formulations of this invention, even when available in higher concentrations than previously envisioned nonetheless are well tolerated, without significant side effect.

All publications and patents mentioned herein are hereby incorporated by reference in their entirety as if each individual publication or patent was specifically and individually indicated to be incorporated by reference. In case of conflict, the present application, including any definitions herein, will control.

While specific embodiments of the subject invention have been discussed, the above specification is illustrative and not restrictive. Many variations of the invention will become apparent to those skilled in the art upon review of this specification and the claims below. The full scope of the invention should be determined by reference to the claims, along with their full scope of equivalents, and the specification, along with such variations.

## EXAMPLES

## SIMULATING KETOROLAC CONTAINING FORMULATIONS FOR SUBCUTANEOUS DELIVERY

## EXAMPLE 1

It was an object to simulate the time course of plasma Ketorolac concentrations and effects with a PatchPump delivery system.

A modified version of the Mandema-Stanski published model for Ketorolac (termed the Modified Mandema-Stanski PK model, or the MMSPK Model) was developed in steps; a base model development step for description of structural components of the model followed by a covariate analysis. For the latter the base model output was used to conduct a graphical evaluation of the covariates at first. A full model including all relevant covariate effects (e.g. each covariate-parameter relationship which was significant during single covariate model testing was included in the full model) was then developed. A final model was chosen by retaining only the statistically significant covariate effects. The parameters in the population models were estimated using the NONMEM software program (version 7.3). The stochastic approximation estimation method (SAEM) was used for estimation. A visual predictive check (VPC) was conducted for the model using all data.

Simulations: Once the model was qualified, stochastic simulations were conducted to assess the expected range of concentrations following several dose regimens. The MMSPK model is a more relevant model in discussing the pharmacokinetics in healthy volunteers following parenteral administration and for making extrapolations to patients. Also, because regimens using PO dosing were of interest, literature values were used for Ka and ALAG for the MMSPK model, assumed to have no BSV.

For the simulations of renally impaired and elderly subjects the clearances reported in the prescribing information were used. This in effect forces the steady state concentrations from SC CIINTCSCI dosing to be identical for the MMSPK model. The distributional and time to steady state aspects of each model will however be different.

**Pharmacokinetic Model Development**

The pharmacokinetic model was developed in the following steps. First, a base model was developed without consideration of covariate effects. For the pharmacokinetic model, a previously developed model was to be used as the base model. Additional evaluations of stochastic models were conducted. Then, a full covariate model was developed by testing covariate parameter relationships graphically and as single covariate models. Covariates that showed a trend or were expected to influence Ketorolac pharmacokinetic (e.g., body size) were tested as single covariate models. The chi-squared test (p<0.01) for the difference in the (objective function value) OBJ between nested models with degrees of freedom equal to the difference in number of parameters between models were used to declare superiority of one model over another. This corresponds to a reduction in OBJ of 7.9 for comparison of models that differ by one parameter. The covariance step was implemented with each NONMEM run, and standard errors for parameter estimates as well as correlation between parameters were evaluated. Models that resulted in parameter estimates with high associated standard error (> 35% of the parameter estimate), models with a high degree of correlation between parameters (> 90%), and models that included a covariate(s) whose effect on the estimated parameter value was negligible (e.g., a less than 20% change in the parameter over the range of covariate values - 5th to 95th percentile - in the database), were not considered further. Covariates that met the criteria for selection were incorporated into a full model. Lastly, the final pharmacokinetic model was chosen using backwards elimination by retaining only the statistically significant (p<0.001) covariate effects. Some predictors were tested in an exploratory fashion but with only 12 healthy male subjects, predictor identification was beyond the scope of this exercise.

**Base Pharmacokinetic Model**

The base pharmacokinetic model consisted of the following components: a structural model that described plasma concentrations of KT as a function of time for the structural model, an interindividual variability (IIV) model that described random variability among individuals in the study population, and a residual error model that characterized the random variability in observed data within an individual. The criteria used for model selection for the pharmacokinetic model are described below, followed by descriptions of the structural, residual error, and IIV model development.

**Model Selection Criteria**

Model selection was based on the following criteria, but not limited to:

Successful achievement of NONMEM minimization and covariance steps indicates that the parameters are all identifiable

- Assessment of standard goodness-of-fit plots
- Reduction in NONMEM OBJ for hierarchical models
- Reductions in IIV and residual variability.

The following diagnostic plots were used in base model selection:

- Population prediction (PRED) and individual prediction (IPRED) versus observations (OBS)
- Individual goodness of fit plots
- CWRES versus PRED and individually weighted residual (IWRES) vs. IPRED
- CWRES versus time
- Histograms and Quantile-Quantile (QQ) plots of CWRES and IWRES to compare it to standard normal distribution
- QQ plots and histograms of IIV.

Population prediction refers to model predictions made with estimates of typical values of structural model parameters, and individual prediction refers to model predictions made with estimates of individual random effects.

**Pharmacokinetic Structural Model**

The MMSPK model was developed as a modification of the MSPK model.

Statistical Modeling

The MMSPK model is a hierarchical model. Random unexplained interindividual variability between subjects (IIV), and within subjects (WSV) was modeled. PK parameters were assumed to be log normally distributed. BSV was described using the following functional form:

$$P_j = TVP \cdot e^{\eta_j} \qquad \text{Equation 1}$$

In this equation $P_j$ is the individual value for the pharmacokinetic parameter in the *jth* individual and $\eta_j$ is an independent random effect with a mean of zero and variance $\omega_P^2$. This function is used for parameters that are ascribed as having a log normal distribution such as clearance.

The residual variability for the pharmacokinetic model was described using a Log Transform Both Sides (LTBS) approach:

$$Ln\left(Cp_{ij}\right) = Ln\left(1000 \cdot Cpred_{ij}\right) + \varepsilon_{ij} \qquad \text{Equation 2}$$

In Equation 2, $Cp_{ij}$ and $Cpred_{ij}$ are the observed and predicted j-th pharmacokinetic response for the i-th subject. The scaling factor of 1000 is needed for changing mg/L to ng/ml. This equation models WSV as additive on the log scale with $\varepsilon_{ij}$ being a random intra-individual effect with mean zero and variance $\sigma^2$.

Estimation Methods

The concentration-time data collected in these studies were analyzed using mixed effects modeling methods as implemented by the computer program NONMEM (version 7, level 3) [Beal SL and Sheiner LB. 1980. The NONMEM System. Am. Stat., 34, 118; Boeckmann A.J., Beal S.L., Sheiner L.B. NONMEM Users Guide - Part III, NONMEM Installation Guide, March 1998, NONMEM Project Group, University of California, San Francisco], compiled using Intel(R) Visual Fortran Intel(R) 64 Compiler Professional for applications running on Intel(R) 64, Version 11.1.065. Diagnostic graphics, exploratory analyses, and post-processing of NONMEM output was performed using SAS, S-Plus, or R version 3.0 or later [SPlus version 6.2 Professional. Copyright (c) 1988-1999. Data Analysis Products Division, MathSoft, Seattle, WA; SAS Institute Inc., SAS 9.2.0 Help and Documentation, Cary, NC: SAS Institute Inc., 2000-2004; R Core Team (2015). R: A language and environment for statistical computing. R Foundation for Statistical Computing, Vienna, Austria].

The MMSPK model used the iterative two-stage (ITS), stochastic approximation expectation maximization algorithm (SAEM), and importance sampling (IMP) methods of estimation, in that order. The covariance step from the IMP method, using the expectation only option (EONLY=1) and the matrix=R option was used to estimate parameter uncertainty. The R option is not available for the SAEM algorithm and the IMP method gives the integrated marginal density. The ADVAN13 differential equation solver was used.

Model Qualification

Model qualification (e.g., evaluation of the stability and predictive performance of the developed final population model) and testing is an important part of model development and is encouraged by the regulatory agencies [Guidance for Industry: Population Pharmacokinetics. U.S. Department of Health and Human Services, Food and Drug Administration, Center for Drug Evaluation and Research (CDER), Center for Biologics Evaluation and Research (CBER), February, 1999; Guideline on Reporting the Results of Population Pharmacokinetic Analyses. CHMP June 2007 http://www.emea.europa.eu/pdfs/human/ewp/18599006enfin.pdf]. The model developed in this analysis was tested and qualified by evaluating the condition number and determining the asymptotic standard errors of the parameter estimates. Standard diagnostic plots were also generated.

Model Stability and Predictability

Model stability was tested through the evaluation of the condition number [10. Primer of Applied Regression and Analysis of Variance. Glantz SA and Slinker BA 1990 McGraw Hill New York NY p225]. A condition number (computed as the square root of the ratio of the largest eigenvalue to the smallest eigenvalue of the correlation matrix) of less than 20 suggests that the degree of colinearity of the parameter estimates is acceptable. A condition number that is in excess of 100 indicates that the model may be unstable due to high colinearity.

Models were selected as appropriate only if the $COV step completed successfully, and the condition number was acceptable indicating that the omega matrix was well conditioned. The predictive validity of the model was examined by comparing the model prediction based on typical parameter values.

One potential problem with population analysis is the tendency of the individual parameter estimates to "shrink" towards the mean value. This shrinkage is usually associated with too little data from each individual to provide a robust individual parameter estimate. When the individual parameter estimates exhibit substantial shrinkage, they no longer reflect the individual pharmacokinetic behavior, which can affect derived parameter estimates such as area under the plasma concentration time curve (AUC). Therefore, in order to evaluate the ability of the present database to provide adequate individual parameter estimates for further derived parameter values, the extent of Bayesian shrinkage was assessed using the method described by Karlsson [Karlsson MO, Model-building diagnostics, DIA Meeting December 2005; Karlsson MO and Savic RM. Diagnosing model diagnostics. Clin Pharmacol Ther 2007: 82(1); 17-20].

Visual Predictive Checks

Visual predictive checks (VPC) [Yano Y, Beal SL, Sheiner LB. 2001. Evaluating pharmacokinetic/pharmacodynamic models using the predictive check. J Pharmacokinet Pharmacodyn, 28(2), 171-92] were conducted to compare the distribution of simulated predictions from both models to those obtained from the original data. In this visual checks, the concentration time profiles were simulated using the final model parameters. One hundred (100) replicates of the study data were simulated. The means, 10th, and 90th percentiles were plotted for both the observed and simulated responses.

Simulation Methods

Simulations were conducted without consideration for parameter precision. An overview of the simulation process is provided in the following sections.

Simulation Overview

For each simulation scenario 800 subjects were simulated. No parameter uncertainty or WSV was incorporated into any of the simulations. BSV was used for both models. The simulation outputs were assessed graphically and summaries of key metrics (see

Table 1) were summarized and tabled. Area parameters were computed using the NONMEM differential equations ($DES) block. Plots were generated to compare non SC route scenarios with SC scenarios. For each plot the 10th, 50th, and 90th percentiles for the simulated pharmacokinetic response was plotted versus time. For each pair of scenarios, plots were generated focusing on an early portion and the steady state portion of therapy. This was done for each model.

Table 1 Description of Derived Parameters

| Parameter | Description |
| --- | --- |
| Cmax | Maximum concentration (ng/ml) |
| Cmin | Minimum concentration (ng/ml) |
| AUC$\tau$ | Area under the curve from 48 - 54 hours (ng·hr/ml) |
| AUC1 | Area under the curve for day 1 (ng·hr/ml) |

(continued)

| Parameter | Description |
|-----------|-------------|
| AUC4 | Area under the curve for day 4 (ng·hr/ml) |
| Cssavg | Average concentration for day 4 (ng/ml) |
| TT370 | Time to reach a concentration of 370ng/ml (hrs) |
| TT1000 | Time to reach a concentration of 370ng/ ml (hrs) |
| PGT5000 | Proportion of subjects with any concentration > 5000ng/ ml |

Parameters from other sources

Because simulations were needed for regimens using oral KT, a Ka of 11/hr and an absorption lag time of 0.2 hrs were obtained from the KT pharmacokinetic review by Mroszczak. That study did not estimate BSV [Mroszczak EJ, Jung D, Yee J, Combs D, Bynum L, Sevelius H, Massey I. Ketorolac tromethamine pharmacokinetics and metabolism after intravenous, intramuscular and oral administration in humans and animals. Pharmacotherapy 10:33S-39S, 1990]. Moreover, elderly subjects and those with renal impairment were of interest. clearance values from the prescribing information [Toradol(R) [package insert]. New York, NY: Pfizer Labs; 2010] were inserted into both models. The MSSPK model was not re-fitted to the training data following these modifications. Final comparisons of the MSPK model incorporated the SC input model developed for the data from continuous infusion studies.

Simulated Dosing Scenarios

The dosing scenarios to be simulated can be divided into two major categories: subjects who are neither elderly nor moderately renally impaired and special populations. The prescribing information [Toradol(R) [package insert]. New York, NY: Pfizer Labs; 2010] defines special populations consisting of patients older than 65 years old, patients with moderately elevated creatinine, and patients under 50 kg (110 pounds).

Normal Renal Function

The first category is the dosing scenarios for the subjects with normal renal function. For this group, several approaches to dosing were modeled and are further described in

Table 2.

Two of the scenarios (30 mg IM q6h and 30 mg IM followed by 10 mg PO q6h) represent currently approved indications. The third scenario (30 mg IM co-administered at the start of a 90 mg/day CSCI administration) represents a possible dosing scenario with continuous infusion. A fourth scenario uses a 15 mg IM load followed by 100 mg/day CSCI.

Table 2 Scenarios for Subjects with Normal Renal Function

| Regimen | Description |
|---------|-------------|
| P010IM30 | One 30 mg IM dose then 10 mg PO q6h for 4 days.<br>This simulation represents the lowest exposure to KT expected for otherwise normal subjects, based on the indication of always initiating with parenteral KT (30 mg IM) and then quickly transitioning to PO administration (10 mg PO q6h). |
| IM30 | 30 mg IM q6h for 4 days. |
| | This simulation represents the highest exposure to KT expected for otherwise normal subjects, based on the indication of initiating with parenteral KT (30 mg IM) and then staying with parenteral (30 mg IM q6h). |
| SC100IM15 | One 15 mg IM dose then 100 mg/day SC CSCI for 4 days. |
| SC90IM30 | One 30 mg IM dose then 90 mg/day SC CSCI for 4 days. |

(continued)

| Regimen | Description |
|---|---|
|  | This simulation represents one of the target configurations or indications for continuous infusion. In this scenario, treatment is initiated with a 30 mg IM injection and co-administered with continuous infusion KT at 90 mg/day. With this scenario, the total dose of KT on the first day (120 mg) is the same as the daily dose for the all IM dosing scenario. |

Elderly or Impaired Renal Function

The other category is the dosing scenarios for the subjects who are elderly or have impaired renal function. It should be noted that the dose recommendations in the prescribing information recommend the same dose adjustments for subjects whose weight is less than 50 kg [Toradol(R) [package insert]. New York, NY: Pfizer Labs; 2010]. For this group, four approaches to dosing were modeled and are further described in

Table 3.

Two of the scenarios (15 mg IM q6h and 15 mg IM followed by 10 mg PO q6h) represent currently approved dosing regimens for this sub-population. The other scenario (15 mg IM co-administered at the start of a 45 mg/day CSCI administration) represents a possible dosing scenario with continuous infusion.

Table 3 Dosing Scenarios for Elderly (Age ≥ 65yrs) or Moderately Renally Impaired Subjects (SCR≥ 1.9mg/dL).

| Regimen | Description |
|---|---|
| P010IM15 | One 15 mg IM dose then 10 mg PO q6h for 4 days.<br>This simulation represents the lowest exposure to KT expected for this sub-population, based on the prescribed dosing information to always initiate therapy with a lower dose of parenteral KT (15 mg IM) and then quickly transitioning to PO administration (10 mg PO q6h). |
| IM15 | 15 mg IM q6h for 4 days. |
|  | This simulation represents the highest exposure to KT expected for this subpopulation, based on the indication of initiating with a lower dose (half of maximum dose for non impaired patients) of parenteral KT (15 mg IM) and then staying with that lower parenteral dose (15 mg IM q6h). |
| SC45IM15 | One 15 mg IM dose then 45 mg/day SC CSCI for 4 days.<br>This simulation represents the target dosing regimen for continuous infusion for this subpopulation based on the USPI dosing which is half the dose prescribed of non impaired patients.. In this scenario, treatment is initiated with a 15 mg IM injection and co-administered with continuous infusion KT at 45 mg/day. With this scenario, the total dose of KT on the first day (60 mg) is the same as the daily dose for the all IM dosing scenario. |

Archival and Systems qualifications

All data sets, control streams figures and tables generated during the course of this analysis are archived at both Projections Research, Inc., Phoenixville, PA. If requested, a copy of these archives can be provided by CD or other archival method.

Systems qualifications for NONMEM were conducted at Projections Research Inc. Vendor distributed control streams and data files were run and crosschecked with provided listings. Additional test kits were generated to ensure proper handling of user generated subroutines and non-standard applications.

R has been qualified but not validated.

## RESULTS

In these simulations, pain relief is obtained more quickly by administration of an intramuscular loading dose followed immediately by use of the PatchPump device.

The MMSPK model, provides a high CL estimate (2.92 L/hr) and estimated Ka for IM absorption (11.6/hr).

The overall goal of the pharmacokinetic simulations reported here was to determine if a dosing regimen could be found that would provide a pharmacokinetic profile with blood levels between the approved IM and PO dosing regimens, thereby indicating that continuously infused Ketorolac can provide adequate analgesia while maintaining an acceptable safety profile for treating acute post-operative pain.

In non-elderly, non-renally impaired patients, the maximum dose in the prescribing information is 30 mg IM q6h for no more than 5 days. The approved regimen that would result in the lowest concentrations and overall exposure is 10 mg PO q6h (preceded by one IM dose) every 6 hours, not to exceed 40 mg/day and for no more than 5 days. Thus if the concentrations of Ketorolac following an IM loading dose given simultaneously with the start of a continuous subcutaneous infusion via continuous infusion achieves concentrations that are bracketed on the high side by a regimen of 30 mg IM q6h and on the low side by 10 mg PO q6h, then analgesia with an acceptable safety profile should be achieved in post-operative patients with continuous infusion.

The results of the simulations described above suggest that if a loading dose of 30 mg IM and a continuous subcutaneous infusion of 90 mg/day were tested in healthy volunteers that the goal of achieving the desired concentrations within the brackets noted above would be achieved. Table A shows a summary of the derived exposure parameters for normal subjects for both models. An alternative dosing regimen of 15 mg loading and 100 mg/day with continuous infusion was also evaluated.

**Table A Summary of Key Derived Pharmacokinetic Exposure Parameters for 'Normal' Subjects**

| Dose/MSPK | AUC4 | Cssavg |
|---|---|---|
| IM30+PO10 /MMSPK | 9392 (6476.5 13854) | 391.3 (269.9 577.2) |
| IM30 /MMSPK | 28127 (19417 41337) | 1172 (809 1722.4) |
| IM30SC90 /MMSPK | 20344.5 (13853 30417.5) | 847.7 (577.2 1267.4) |
| Units are ng/ml or ng*hr/ml, AUC=area under the curve, AUC4 is AUC for day 4, Cssavg = average conc for day 4. | | |

The time to concentrations of 370 ng/ml and 1000 ng/ml are within 0.1 hours so that onset of action would be fast. Also the risk of a subject having a concentration above 5000 ng/ml is very small, 0 to 1 out of 800 simulated subjects.

The dosing simulations in the special populations of the renally impaired and the elderly give qualitatively similar results to the non-impaired subjects such that dosing at a 50% reduction in the subcutaneous infusion rate achieves exposures between the high IM dose and the low PO dose. As can be seen for the summary of the renally impaired subjects (Table B), the proposed dose regimen maintains exposures that are bracketed by exposures that would be achieved using the dose recommendations for IM or PO. The 50% decrease in dose is consistent with current prescribing information. The same is true for elderly subjects (Table C).

**Table B Summary of Key Derived Pharmacokinetic Exposure Parameters for 'Moderately Renally Impaired' Subjects**

| Dose/Model | AUC4 | Cssavg |
|---|---|---|
| IM15+PO10 /MMSPK | 25322.5 (17827.5 35793) | 1055.1 (742.8 1491.4) |
| IM15 /MMSPK | 37737 (26680.5 53104.5) | 1572.4 (1111.7 2212.7) |
| IM15+SC45 /MMSPK | 27593 (19146.5 39576.5) | 1149.7 (797.8 1649) |
| Units are ng/ml or ng*hr/ml, AUC=area under the curve AUC4 is AUC for day 4, Cssavg = average conc for day 4. | | |

**Table C Summary of Key Derived Pharmacokinetic Exposure Parameters for 'Elderly' Subjects**

| Dose/Model | AUC4 | Cssavg |
|---|---|---|
| IM15+PO10 /MMSPK | 20303 (14137 29173) | 846 (589 1215.5) |
| IM15 /MMSPK | 30320 (21179.5 43422) | 1263.3 (882.5 1809.2) |
| IM15+SC45 /MMSPK | 22059.5 (15161.5 32337) | 919.1 (631.71347.4) |
| Units are ng/Mml or ng*hr/ml, AUC=area under the curve, AUCτ = AUC from 48 to 54hrs, AUC1 is AUC for day 1, AUC4 is AUC for day 4, Cssavg = average conc for day 4. | | |

In the simulations for non-elderly subjects with otherwise normal renal function, the following results were observed:

- The times to reach concentrations ≥ 1000 ng/ml were shorter for the 30 mg IM + 90 mg/day CSCI regimen than with the 15 mg IM + 100 mg/day regimen.

- The 30 mg IM + 90 mg/day CSCI regimen attained steady state concentrations bounded between the peaks and troughs simulated with the 30 mg IM q6h regimen.

- The 30 mg IM + 90 mg/day CSCI regimen attained steady state concentrations close to or above the peaks simulated with the 30 mg IM + 10mg PO q6h regimen.

- These observations indicate that 30 mg IM + 90 mg/day CSCI avoids extreme concentrations associated with toxicity and attains concentrations similar to the average concentrations seen with IM dosing quickly.

In the simulations for the subpopulation of elderly or impaired renal function, the following results were observed:

- All regimens used a 15 mg IM load and took at least 18 minutes to attain concentrations ≥ 1000 ng/ml.

- The 15 mg IM + 45 mg/day CSCI regimen attained steady state concentrations approximately equal to the troughs seen with 15 mg IM q6h dosing.

- The 15 mg IM + 45 mg/day CSCI regimen attained steady state concentrations bounded between the peaks and troughs simulated with the 15mg IM + 10mg PO q6h regimen.

These observations indicate that the 45 mg/day CSCI regimen is similar to the 15mg IM 10 mg PO q6h dosing. This is expected at steady state given the comparable daily dosing rates (40 and 45 mg/day) for these regimens.

## KETOROLAC CONTAINING FORMULATIONS FOR SUBCUTANEOUS DELIVERY

### EXAMPLE 2

Embodied compositions are prepared containing the following:

Keterolac tromethamine: 60, 90 and 120 mg/mL and other various increments
Sodium ascorbate: 0-2 mg/ml
Sodium phosphate buffer: 0-2 mg/ml
Ethanol: 0-20%
1N HCl or 1N NaOH, USP:As needed for pH adjustment [to achieve a neutral pH]
Water for injections: As needed per batch size

In some aspects, the formulation is a non-ethanolic formulation.
The formulation is sterile filtered, aseptically filled into pre-sterilized unit dose containers, and stoppered, prior to assembly within an integrated delivery system.

## COMPARISON OF CONTINUOUS SUBCUTANEOUS DELIVERY VERSUS REPEATE INTRAMUSCULAR BOLUS INJECTIONS OF KETOROLAC CONTAINING FORMULATIONS IN HUMAN PATIENTS

### EXAMPLE 3

### Ethics

The design and monitoring of this clinical study complied with the ethical principles of Good Clinical Practice, in accordance with the Declaration of Helsinki. Study protocol and informed consent documents were reviewed and approved by the Institutional Review Board (IRB) Chesapeake Research Review, Inc., and informed consent was obtained from all subjects before participation. The study was conducted at one site. Prior to study initiation, all pertinent study documents were reviewed by the IRB.

**Study Design and Conduct**

The clinical conduct, clinical 1ab testing and sample analysis were conducted by Celerion in Lincoln, NE. The study design is presented schematically in Figure 1.

This study was an open-label, randomized, 2-period, 2-way crossover in healthy subjects. Twelve (12) subjects were randomized to receive a total daily dose of 120 mg Ketorolac tromethamine (Ketorolac Tromethamine Injection, USP 30 mg/mL, manufactured by Hospira Inc., Lot No.: 51600LL) administered as either: (Treatment A) one continuous SC infusion of 4 mL to the abdomen over 24 hours at a steady rate of 5 mg/hr (0.167 mL/hr), or (Treatment B) four IM bolus injections of 30 mg (1 mL) to alternating gluteus maximus muscle every 6 hours. For the continuous SC infusion, the MedFusion 3500 syringe pump was assembled with Animas Inset Infusion Set 23 inch 6mm 10/box Grey (100-182-00), B-D Luer-Lok Syringe 5mL and B-D 20 G 1 inch needle (Becton Dickinson). The treatments were separated by a washout period of $\geq$ 4 days. The total daily dose of 120 mg Ketorolac tromethamine administered as 4 x 30 mg/mL IM bolus injections is a recommended treatment for subjects under 65 years of age according to the product labeling. 2 The same dose has been reported to be well tolerated when administered in a continuous SC infusion regimen to patients in several studies, and as such was chosen for this study. 1

Blood samples for the determination of plasma Ketorolac following continuous SC infusion (Treatment A) were collected at the following time points: pre-dose (0 hour), 0.25, 0.5, 1, 1.5, 2, 4, 6, 8, 10, 12, 14, 16, 18, 20, 22, 23, 24 (2 blood samples were collected at the 24 hour time point; 1 immediately prior to the end of infusion and 1 immediately following end of infusion), 24.167, 24.33, 24.5, 25, 26, 28, 30, 32, 36, and at 48 hours post-dose. The time for post-dose blood collection are expressed relative to the start of infusion.

Blood samples for the determination of plasma Ketorolac following IM injections (Treatment B) were collected at the following time points: pre-dose (0 hour), 0.167, 0.25, 0.5, 0.75, 1, 1.25, 1.5, 2, 3, 4, 6 (pre-dose), 6.25, 6.5, 6.75, 7, 8, 10, 12 (pre-dose), 12.25, 12.75, 13, 14, 16, 18 (pre-dose), 18.25, 18.5, 18.75, 19, 20, 22, 24, 28, 32, and at 48 hours post-dose. The time for post-dose blood collection are expressed relative to the first IM injection.

All subjects underwent pre-dose (each period) and post-study assessments including physical examinations, routine laboratory tests, vital signs, and 12-lead electrocardiograms (ECGs). Alcohol and drug screen were conducted before each dosing period. All subjects were between 19 and 55 years old with a body mass index (BMI) between 18.5 and 30.0 kg/m2. Subjects had to refrain from the use of any prescription medications including over-the-counter products, herbal products or vitamin supplement within 14 days of dosing.

Subjects were excluded from participation in the study if any clinically significant medical or psychiatric conditions or disease or illness were found that would potentially confounded the results of the study including but not limited to: cardiovascular disease, chronic renal failure, disorders of cerebral or peripheral perfusion, seizures, peptic ulcer, abnormal or excessive bleeding, asthma, or allergic reaction to aspirin. Subjects were also excluded if they had a positive drug screen, positive urinary cotinine test, had a creatinine clearance < 90 ml/min or if they had any tattoo(s), scarring or any abdominal skin abnormalities at or near the site of injection which may interfere with injection/infusion site examination(s).

**Analytical Methods**

Ketorolac plasma concentrations were determined using a validated liquid chromatographic tandem mass spectrometry (LC-MS/MS) method, with a lower limit of quantitation (LLOQ) of 10.0 ng/mL9. The validated plasma Ketorolac concentration range was 10.0 to 5000 ng/mL. Assay performance was monitored using quality control (QC) samples at concentrations of 30.0 ng/mL, 200 ng/mL, and 3800 ng/mL; the %CV for the QCs ranged from 1.3 % to 15.1 % and accuracy was 91.6 - 102.5%.

**PK Parameter Estimates**

Non-compartmental PK parameters were calculated from Ketorolac concentrations-time data in plasma using Phoenix® WinNonlin® Professional (Versions 6.3; Pharsight, Mountain View, California). These included: area under the concentration-time curve (AUC) from time 0 to the time of the last measurable concentration ($AUC_0$-last), AUC from time 0 to the 24-hour time point ($AUC_0$-24), AUC from time 0 to infinity ($AUC_0$ inf; treatment A only), percentage of the area extrapolated for calculation of $AUC_0$-inf (AUCext; treatment A only), AUC over the final dosing interval (tau) ($AUC_{tau}$; treatment B only), average concentration calculated as the ratio of $AUC_{tau}$ to tau ($C_{avg}$; treatment B only), plasma trough (predose) concentration observed at the end of the dosing interval (Ctrough treatment B only), concentration at steady-state (Css), maximum observed concentration ($C_{max}$), time to $C_{max}$ (tmax), apparent total body clearance (CL/F), and apparent volume of distribution (Vz/F), apparent first order terminal elimination constant (kel), half-life (t½). $AUC_0$-inf was calculated as the sum $AUC_0$-last plus the ratio of the last measurable plasma concentration (Clast) to kel; $AUC_0$-last, $AUC_0$-24, and $AUC_{tau}$ were calculated by the linear trapezoidal summation method; AUCext was calculated as 1-

$AUC_0$-last/$AUC_0$-inf multiplied by 100, $C_{max}$, $C_{trough}$, $t_{max}$ were reported as observed values; $C_{avg}$ was calculated as $AUC_{tau}$ divided by 6 hours for the IM administration; Css was calculated as the average of the concentrations from the time steady-state was reached until the end of SC infusion; CL/F was calculated as dose/$AUC_0$-inf (for SC administration) or dose/$AUC_{tau}$ (for IM administration); Vz/F was calculated as (CL/F)/kel); kel was calculated by linear least-square regression analysis using the maximum number of points in the terminal log-linear phase (using 3 or more non-zero plasma concentrations); t½ was calculated as ln(2)/kel.

**Statistical Analyses**

Statistical analyses were conducted to compare Ketorolac $AUC_0$ 24, $AUC_0$ last, and $C_{max}$ following continuous SC infusion and repeat IM bolus injections. A SAS® mixed model procedure was performed on the natural log (ln)-transformed PK parameters. The mixed model included sequence, treatment, and period as fixed effects and subject nested within sequence as a random effect. The mixed model included calculation of the least-squares means the difference between geometric mean, and the standard error associated with this difference. The 90% confidence intervals (CIs) for the ratios were derived by exponentiation of the CIs obtained for the difference between treatment LSM resulting from the analyses on the ln transformed $AUC_0$ 24, $AUC_0$-last, and $C_{max}$. The CIs were expressed as a percentage relative to the reference treatment (repeat IM bolus injections).

The steady-state analysis was performed using the Helmert contrast, where the ln-transformed predose Ctrough concentrations at 6, 12, 18, and 24 hours for the repeat IM injections treatment and the ln transformed concentrations at the 4-, 6-, 8-, 10-, 12-, 14-, 16-, 18-, 20-, 22-, 23-, and 24 hour (prior to end of infusion) time points for the SC infusion treatment were utilized. The ANOVA model included period and time as fixed effects. Geometric mean and p-values of the contrast were reported. Prior to estimating the fixed-effects and the contrasts, an appropriate covariance structure was selected using Akaike's information criterion (AIC). In the event that no standard compact covariance structure would be appropriate, an unstructured covariance parameterization would be used. The statistical analysis was done using the SAS® Mixed procedure. Helmert contrasts were constructed such that each time point was compared to the mean of the subsequent time point. Steady-state was concluded at the time point where no more statistical differences (alpha=5%) were observed. For Treatment A, time to steady-state (Tss) was defined as the time point where no more statistical differences (alpha=5%) were observed. In addition, spline (quadratic plateau) regression was used for the estimation of individual's time to steady-state based on individual plasma concentrations (from 4 to 24 hours), using SAS NLIN procedures. 10

**RESULTS**

**Demographic**

Baseline demographics of the participants are shown in Table 1. A total of 12 subjects were enrolled, and 11 completed the study. One subject was discontinued from the study prior to dosing in Period 2 due to several AEs (diarrhea, nausea, and vomiting) that were considered not related to study drug.

**Table 1 Demographic Summary**

| Trait | Category/ Statistic | Treatment Sequence | | Overall |
| | | AB | BA | |
|---|---|---|---|---|
| Gender | Male | 6 (100%) | 6 (100%) | 12 (100%) |
| Race | Black or African American | 2 (33%) | 0 (0%) | 2 (17%) |
| | White | 4 (67%) | 6 (100%) | 10 (83%) |
| Age (yrs) | N | 6 | 6 | 12 |
| | Mean (SD) | 31.5 (12.2) | 33.3 (12.0) | 32.4 (11.6) |
| | Minimum, Maximum | 19, 54 | 19,46 | 19, 54 |
| Weight (kg) | N | 6 | 6 | 12 |
| | Mean (SD) | 80.8 (13.5) | 78.3 (13.0) | 79.6 (12.7) |
| | Minimum, Maximum | 62.2, 95.6 | 61.3,99.2 | 61.3,99.2 |
| Height (cm) | N | 6 | 6 | 12 |

(continued)

|  | | Treatment Sequence | | |
|---|---|---|---|---|
| Trait | Category/ Statistic | AB | BA | Overall |
|  | Mean (SD) | 180.5 (9.7) | 178.0 (10.0) | 179.3 (9.5) |
|  | Minimum, Maximum | 171, 197 | 167, 195 | 167, 197 |
| BMI (kg/m$^2$) | N | 6 | 6 | 12 |
|  | Mean (SD) | 24.76 (3.23) | 24.47 (1.51) | 24.61 (2.41) |
|  | Minimum, Maximum | 21.28, 29.60 | 21.94, 26.14 | 21.28, 29.60 |
| Treatment A = 120 mg Ketorolac tromethamine administered as a continuous SC infusion over 24 hours<br>Treatment B = 120 mg Ketorolac tromethamine administered as 4 IM bolus injections of 30 mg every 6 hours<br>Age is calculated at the time of dosing. Weight, height, and BMI at Screening were summarized. | | | | |

**Pharmacokinetic**

Mean plasma concentrations and PK parameters for Ketorolac are provided in Figure 2 and Table 2, respectively.

**Table 2 Summary of Ketorolac Pharmacokinetic Parameters Following 120 mg Ketorolac Tromethamine Administered as a Continuous SC Infusion Over 24 Hours (Treatment A) and Four IM Bolus Injections of 30 mg Every 6 Hours (Treatment B)**

| Pharmacokinetic Parameters | Treatment A<br>N = 11 | Treatment B<br>N = 12 |
|---|---|---|
| $AUC_{0\text{-}last}$ ($\mu$g*hr/mL)[a] | 28.48 (30.9) | 27.83 (29.8) |
| $AUC_{0\text{-}24}$ ($\mu$g*hr/mL)[a] | 21.54 (25.2) | 23.57 (24.7) |
| $AUC_{0\text{-}inf}$ ($\mu$g*hr/ML)[a] | 29.06 (31.8) | - |
| $AUC_tAU$ ($\mu$g*hr/mL)[a] | - | 6.813 (27.1) |
| $AUC_{\%extrap}$ (%)[b] | 1.96 $\pm$ 1.2860 | - |
| $C_{max\_all}$ ($\mu$g/mL)[a] | 1.344 (25.6) | 2.240 (17.6) |
| $C_{max0\text{-}6}$ ($\mu$g/mL)[a] | - | 2.039 (17.9) |
| $C_{max6\text{-}12}$ ($\mu$g/mL)[a] | - | 1.952 (21.4) |
| $C_{max12\text{-}18}$ ($\mu$g/mL)[a] | - | 1.955 (26.4) |
| $C_{max18\text{-}24}$ [$\mu$g/mL][a] | - | 2.049 (19.3) |
| $C_{avg}$ [$\mu$g/mL][a] | - | 1.136 (27.1) |
| $C_{ss}$ ($\mu$g/mL)[b] | 1.238 $\pm$ 0.29960 | - |
| $t_{max\_all}$ (hr)[c] | 23.0 (12.0, 24.2) | 9.25 (0.249, 19.0) |
| $t_{max0\text{-}6}$ (hr)[c] | - | 0.5000 (0.249, 1.25) |
| $t_{max6\text{-}12}$ (hr)[c] | - | 6.250 (6.25, 6.75) |
| $t_{max12\text{-}18}$ (hr)[c] | - | 12.25 (12.2, 13.0) |
| $t_{max18\text{-}24}$ (hr)[c] | - | 18.50 (18.3, 19.0) |
| $t_{1/2}$ (hr)[b] | 6.93 $\pm$ 0.84 | 7.02 $\pm$ 1.44 |
| $K_{el}$ (1/hr)[b] | 0.1014 $\pm$ 0.01213 | 0.1029 $\pm$ 0.02289 |
| CL/F (L/hr)[b] | 2.924 $\pm$ 0.97994 | 3.083 $\pm$ 0.89568 |
| Vz/F (L)[b] | 29.10 $\pm$ 10.912 | 30.26 $\pm$ 7.6941 |
| Treatment A: 120 mg Ketorolac tromethamine administered as a continuous SC infusion over 24 hours. | | |

(continued)

| Pharmacokinetic Parameters | Treatment A N = 11 | Treatment B N = 12 |
|---|---|---|
| Treatment B: 120 mg Ketorolac tromethamine administered as four IM bolus injections of 30 mg every 6 hours. a: Presented as geom. mean (geom. CV%) b: Presented as mean ± SD c: Presented as median (minimum, maximum) $C_{max}$ and $T_{max}$ presented for Treatment B are calculated over the whole sampling interval | | |

Overall exposure of Ketorolac, as measured by $AUC_{0-24}$ and $AUC_{0-last}$, were similar following continuous SC infusion and repeated IM bolus injections of Ketorolac tromethamine. Ketorolac $C_{max}$ was 40% lower following continuous SC infusion compared to following 4 IM bolus injections. The Ketorolac mean half-life (~ 7 hours) was comparable across treatments. Following treatments A and B respectively, CL/F was 2.924 ± 0.97994 L/hr and 3.083 ± 0.089568 L/hr and Vz/F was 29.10 ± 10.912 μL and 30.26 ± 7.6941 L.

Statistical comparisons of Ketorolac PK are presented in Table 3.

**Table 3 Summary of the Statistical Comparison of Ketorolac Pharmacokinetic Parameters Following 120 mg Ketorolac Tromethamine Administered as a Continuous SC Infusion over 24 Hours and Four IM Bolus Injections of 30 mg Every 6 Hours (Treatment A Versus Treatment B)**

| Parameter | Geometric LSM | | Geometric Mean Ratio (%) | 90% Confidence Intervals | Intrasubje ct %CV |
|---|---|---|---|---|---|
| | Treatment A (Test, N = 11) | Treatment B (Reference, N = 12) | | | |
| $AUC_{0-24}$ (μg*hr /mL) | 21.51 | 23.58 | 91.23 | 86.9-95.78 | 6.22 |
| $AUC_{0-last}$ (μg*hr /mL) | 28.26 | 27.83 | 101.53 | 96.33-107.02 | 6.72 |
| $C_{max\_all}$ (μg/mL) | 1.341 | 2.240 | 59.86 | 56.2-63.76 | 8.08 |
| Treatment A: 120 mg Ketorolac tromethamine administered as a continuous SC infusion over 24 hours. Treatment B: 120 mg Ketorolac tromethamine administered as four IM bolus injections of 30 mg every 6 hours. Parameters were ln-transformed prior to analysis. Geometric least-squares (LS) means were calculated by exponentiating the LS means from the mixed-effects model. % Geometric Mean Ratio (GMR)= 100*(test/reference) Intrasubject CV% = 100 x sqrt[exp(residual variance)-1] MSE = Residual variance from the mixed-effects model | | | | | |

Geometric LSM values for plasma Ketorolac $AUC_{0-24}$ and $AUC_0$ last were similar following each treatment. The Geometric mean ratio (Treatment A/Treatment B) was 91.23% (90% CI: 86.9, 95.78) for $AUC_{0-24}$ and 101.53% (90% CI: 96.33, 107.02) for $AUC_{0-last}$). $C_{max}$ was lower following treatment A. The Geometric LSM values were 1.341 μg/ml and 2.240 μg/ml; the geometric mean ratio (Treatment A/Treatment B) for $C_{max}$ was 59.86 (90% CI: 56.2, 63.76)

Steady-state assessments are presented in Table 4 to Table 6.

**Table 4 Steady-State Assessment of Plasma Ketorolac Concentrations Following 120 mg Ketorolac Tromethamine Administered as Continuous SC Infusion Over 24 Hours (Treatment A)**

| Hour | Geometric LSM (ng/mL) | p-Value |
|---|---|---|
| Hour 4 | 617.062 | <.0001 |
| Hour 6 | 717.645 | <.0001 |
| Hour 8 | 837.573 | <.0001 |
| Hour 10 | 894.499 | 0.0205 |

(continued)

| Hour | Geometric LSM (ng/mL) | p-Value |
|---|---|---|
| Hour 12 | 946.433 | 0.0011 |
| Hour 14 | 1018.532 | 0.0033 |
| Hour 16 | 1046.459 | 0.0014 |
| Hour 18 | 1159.498 | 0.3456 |
| Hour 20 | 1208.301 | 0.8428 |
| Hour 22 | 1196.251 | 0.9488 |
| Hour 23 | 1226.657 | 0.3931 |
| Hour 24 | 1173.230 | |

Concentrations were In-transformed prior to analysis.

Geometric least-squares means (LSMs) were obtained by taking exponential of the LSMs from ANOVA.

p-value corresponds to the Helmert contrast, i.e., the comparison of that hour versus the average of the remaining hours.

**Table 5 Summary of the Individual Steady-State Assessment of Plasma Ketorolac Concentrations Following 120 mg Ketorolac Tromethamine Administered as Continuous SC Infusion Over 24 Hours (Treatment A)**

| | Time to Steady-State (hr) | Plateau Concentration (ng/mL) |
|---|---|---|
| N | 11 | 11 |
| Mean (SD) | 6.30 (4.17) | 1053 (234.5) |
| CV (%) | 66.3 | 22.3 |
| Median (Min, Max) | 3.68 (1.98, 13.63) | 1084 (572.4, 1426) |

Concentrations were In-transformed prior to analysis.

Spline (Quadratic Plateau) Regression was used for individual steady-state analysis.

The analysis was performed using SAS NLIN procedure.

**Table 6 Steady-State Assessments of Plasma Ketorolac Concentrations Following 120 mg Ketorolac Tromethamine Administered as Four IM Bolus Injections Every 6 Hours (Treatment B)**

| Hour | Geometric LSM | p-Value |
|---|---|---|
| Hour 6 | 302.545 | 0.0003 |
| Hour 12 | 368.216 | 0.0017 |
| Hour 18 | 432.393 | 0.0004 |
| Hour 24 | 564.178 | . |

Concentrations were In-transformed prior to analysis.

Geometric least-squares means (LSMs) were obtained by taking exponential of the LSMs from ANOVA.

p-value corresponds to the Helmert contrast, i.e. the comparison of that hour versus the average of the remaining hours.

Based on Helmert contrast, overall Tss was at 18 hours, with geometric LSM Css of 1160 ng/mL following SC infusion. Based on individual analysis using spline regression, median individual Tss was at 4 hours (ranging from 2 to 14 hours) with corresponding median Css of 1084 ng/mL (ranging from 572 to 1426 ng/mL). Following 4 IM bolus injections, steady-state for plasma Ketorolac concentration was not reached by 24 hours postdose (based on Helmert contrast).

Safety summary

There were no deaths or serious adverse events (SAEs) in this study. One (1) subject was discontinued after completing treatment B due to several AEs (diarrhea, nausea, and vomiting) that were considered not related to study drug. This subject did not receive treatment A. A total of 7 mild treatment emergent adverse events (TEAEs) were experienced by 4 (33%) subjects, 2 subjects following each treatment arm. One subject experienced mild infusion site pain (reported term: abdominal tenderness at infusion site) 13 minutes following the completion of the 24-hour infusion with Treatment A. This event resolved in approximately 2 days and was considered to be possibly related to study drug. Another subject experienced mild injection site hemorrhage (reported term: ecchymosis to left buttock injection site) approximately 6.5 hours after the 4th IM injection. This event resolved in 15 days and was considered not related to study drug but to the injection administration. The remaining AEs were considered unrelated to study treatment. There were no clinically significant trends in vital sign, ECG, or injection/infusion site reaction results.

The PK profiles of Ketorolac tromethamine administered as a continuous SC infusion over a 24-hours period to healthy subjects was compared to an identical total daily dose administered as 4 IM injections given every 6 hours. The results showed that both modes of administration have similar AUCs, suggesting that continuous SC infusion and repeated IM bolus injections have similar relative bioavailability. When Ketorolac tromethamine is administered via continuous SC infusion, the peak plasma concentration is 40% lower compared to when administered as repeat IM bolus injections, which result was not *a priori* expected.

Individual Tss (spline method) ranged from 2.0 to 14 hours with a median time of 4 hours following SC infusion. Following administration of 4 IM bolus injections, steady-state was not attained. Geometric LSM $C_{trough}$ values increased with time. In summary, based on LSM values, the Css following SC infusion was right between the $C_{max}$ and the maximum Ctrough values following IM injections. Based on LSM values, the Css following SC infusion (1160 ng/mL) was between the overall $C_{max}$ (2240 ng/mL) and $C_{trough}$ (564.2 ng/mL at the highest) values following IM injections.

Short-term use of Ketorolac lacks the respiratory depressant effects of opiate analgesics but shares the toxic potentials of other NSAIDs. The major AEs related to Ketorolac tromethamine treatments involve gastrointestinal bleeding, peptic ulcers and perforation of the stomach as well as prolonged platelet aggregations and a decline in renal functions.

In the study reported here, a total dose of 120 mg Ketorolac tromethamine administered via continuous SC infusion appeared to be safe and well tolerated in this study. The steady state concentration following continuous SC infusion was bracketed by the $C_{max}$ and the $C_{trough}$ following IM injection, and this suggests that continuous SC infusion may be a convenient way to achieve post-operative analgesia while avoiding high concentrations and thus may decrease the risk of the serious adverse events. The data presented herein also support the dose-sparing effect of the embodied formulations.

## PREPARATION OF AN EXEMPLIFIED KETOROLAC FORMULATION FOR CONTINUOUS INFUSION

### EXAMPLE 4

The design of a filled primary container for delivery by continuous subcutaneous infusion via body-worn infusion pump device is achieved by means of use of a container that is aseptically filled with a Ketorolac tromethamine formulation as described stoppered with an elastomeric septum and capped with a primary container cap.

The filled primary container is supplied as a pre-filled unit and can be provided in various appropriate at desired product strengths, for example, having a Ketorolac tromethamine concentration of 45 and 90 mg/mL. The fill volume of each unit is approximately 2.65 mL.

Toward this end, in 800 mL of water for injection was dissolved 2 g of Disodium EDTA dihydrate and 90 g of Ketorolac tromethamine, the pH was adjusted to pH 8.0 and water for injection was added to complete the 1 liter volume. The skilled artisan will appreciate that appropriate scale up of the amounts is envisioned, as well.

It will be apparent to those skilled in the art that various modifications and variations can be made to the compositions and processes of this invention. Thus, it is intended that the present invention cover such modifications and variations, provided they come within the scope of the appended claims and their equivalents. The disclosure of all publications cited above is expressly incorporated herein by reference in their entirety to the same extent as if each were incorporated by reference individually.

## Claims

1. Ketorolac for use in a method of providing analgesia, anti-pyretic effects or reducing pain, or reducing administration site irritation, inflammation or a combination thereof, in a subject presenting with a pain condition, the method comprising the steps of:

administering Ketorolac in a sterile fluid composition at a dosage of between **40** mg/mL and **240** mg/mL to said subject and administering said Ketorolac to said subject via continuous subcutaneous delivery.

2. Ketorolac for the use of claim 1, wherein said Ketorolac is Ketorolac tromethamine.

3. Ketorolac for the use of claim 1, wherein said method provides for reducing a dosage of Ketorolac over time.

4. Ketorolac for the use of claim 1, wherein said method provides for sustaining a given dosage of Ketorolac over a prolonged period of time.

5. Ketorolac for the use of claim 4, without adverse effects selected from gastrointestinal bleeding, inhibition of platelet function, renal impairment, or a combination thereof.

6. Ketorolac for the use of claim 1, wherein said method makes use of a selectively activatable body-worn infusion-pump assembly comprising a sealed prefilled drug-reservoir containing a unit dosage form comprising Ketorolac to administer Ketorolac to said subject.

7. Ketorolac for the use of claim 6, wherein said Ketorolac is formulated for single use delivery in a volume not to exceed 2-5 mL, such as a volume not to exceed 3 mL or a volume not to exceed 1 mL.

8. Ketorolac for the use of claim 1, wherein said sterile fluid composition is ethanol free or sodium chloride free.

9. Ketorolac for the use of claim 1, wherein said subject is administered Ketorolac over a period of time of more than 5 consecutive days.

10. Ketorolac for use in a method of providing analgesia, anti-pyretic effects, or reducing pain in a subject in need thereof, where said subject is administered Ketorolac in a sterile fluid composition in a continuous subcutaneous infusion at a rate of about 90 mg per 24 hours over period of 24 to 120 hour; or is administered Ketorolac in a sterile fluid composition at a dosage of about 30 mg subcutaneously in a period not exceeding 1 hour followed by a continuous subcutaneous infusion of 90 mg of Ketorolac over the remaining 24 hours, followed by a continuous subcutaneous infusion of Ketorolac at a rate of 120 mg per 24 hours over an additional period of 24 to 96 hours; or is administered a bolus of Ketorolac in a sterile fluid composition at a dosage of about 30 mg intramuscularly or intravenously followed by a continuous subcutaneous infusion of 90 mg of Ketorolac over the first 24 hours, followed by a continuous subcutaneous infusion of Ketorolac at a rate of 120 mg per 24 hours over a period of 24 to 96 hours.

11. A drug infusion pump loaded with an amount of Keterolac providing for a reduced dosage than typically provided, wherein said Ketorolac is in a sterile fluid composition and said Ketorolac is present at a concentration of between **40** mg/mL and **240** mg/mL formulated for single use delivery in a volume not to exceed 1-10 mL, wherein the drug infusion pump is programmed to deliver the Keterolac via continuous subcutaneous delivery.

12. The drug infusion pump of claim 11 wherein the said drug infusion pump is a body-worn infusion pump.

13. The drug infusion pump of claim 12 wherein the said body-worn infusion pump is a pre-filled and preprogrammed pump.

14. The drug infusion pump of claim 11, 12 or 13 that is preprogrammed to administer Ketorolac according to the method defined in claims 1 to 10.

15. A drug infusion pump loaded with an amount of Keterolac providing for a reduced dosage than typically provided, wherein said Keterolac is in a sterile fluid composition and said Keterolac is present at a concentration of between **40** mg/mL and **240** mg/mL formulated for single use delivery in a volume not to exceed 1-10 mL, wherein the said drug infusion pump delivers the Keterolac via continuous subcutaneous delivery.

16. The drug infusion pump of claim 15 that is a pre-filled, pre-programmed pump.

17. The pre-filled, pre-programmed pump of claim 16, wherein said pre-filled, pre-programmed pump is programmed to deliver Ketorolac at a dosage of between about **40** - **120** mg/day.

**18.** The pre-filled, pre-programmed pump form of claim 17, wherein said sterile fluid composition is ethanol-free.

**19.** The pre-filled, pre-programmed pump form of claim 18, wherein said Ketorolac is present at a concentration of between **120** mg/mL and **240** mg/mL.

**20.** The pre-filled, pre-programmed pump of claim 17, wherein said composition provides for a maximal volume of infusion which does not exceed 5 mL for single use.

**21.** The pre-filled, pre-programmed pump of claims 11 to 20, wherein said composition comprises Disodium EDTA dihydrate as the preservative in said dosage form.

**22.** The pre-filled, pre-programmed pump of claims 11 to 21, wherein said composition is at a pH of about from 7.6 to about 8.0.

**23.** The pre-filled, pre-programmed pump of claims 11 to 22, wherein said composition provides for a maximal volume of infusion which does not exceed 1 mL for single use.

**24.** The pre-filled, pre-programmed pump of claim 11, wherein said Ketorolac is provided to said subject at a rate of less than 4 mL/day.

**25.** The drug infusion pump of claims 11 to 24, wherein said Ketorolac is Ketorolac tromethamine.

**26.** A unit dosage form of Ketorolac providing for a reduced dosage than typically provided, wherein said dosage form of Ketorolac is in a sterile fluid composition formulated for continuous subcutaneous delivery via pre-filled, pre-programmed pump assembly, and wherein said Ketorolac is present at a concentration of between 40 mg/mL and 240 mg/mL formulated for single use delivery in a volume not to exceed 1-10 mL, wherein the reduced dosage of Ketorolac provides a PK profile with Ketorolac blood levels between predetermined IM and PO dosing regimens based on simulation results from a MMSPK model at a dosage of less than 120 mg/day that provides effective analgesia, antipyretic effects or reduction of pain.

**27.** A unit dosage form of Ketorolac in a sterile fluid composition formulated for continuous subcutaneous delivery via pre-filled, pre-programmed pump pump assembly, wherein said Ketorolac is present at a concentration of between **40** mg/mL and **240** mg/mL formulated for single use delivery in a volume not to exceed 1-10 mL, wherein said composition is alcohol free, sodium chloride free, or a combination thereof, at a pH of about 7.5 - 8.5 and stable for a period of at least 12 months at room temperature.

**28.** The unit dosage form of claim 26 or 27, wherein said composition contains only a single preservative, which preservative is EDTA.

Treatment A: 120 mg ketorolac tromethamine administered as a continuous SC infusion over 24 hours.
Treatment B: 120 mg ketorolac tromethamine administered as four IM bolus injections of 30 mg every 6 hours.

**Figure 1**

**Figure 2**

Europäisches
Patentamt

European
Patent Office

Office européen
des brevets

**EUROPEAN SEARCH REPORT**

Application Number

EP 18 15 0895

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/087658 A2 (SUN PHARMA ADVANCED RES CO LTD [IN]; KHOPADE AJAY JAYSINGH [IN]; HALDE) 16 July 2009 (2009-07-16) | 26-28 | INV.<br>A61K31/407<br>A61P29/00<br>A61K9/00 |
| Y | * page 3, line 9 - line 12 *<br>* page 5, line 28 - line 29 *<br>* page 7; example 1 * | 1-9, 11-28 | A61K9/08<br>A61M5/142 |
| X | DE CONNO F ET AL: "Tolerability of ketorolac administered via continuous subcutaneous infusion for cancer pain: A preliminary report",<br>JOURNAL OF PAIN AND SYMPTOM MANAGEMENT,<br>ELSEVIER, NEW YORK, NY, US,<br>vol. 9, no. 2,<br>1 February 1994 (1994-02-01), pages 119-121, XP023115617,<br>ISSN: 0885-3924, DOI:<br>10.1016/0885-3924(94)90164-3<br>[retrieved on 1994-02-01] | 1-28 | |
| Y | * abstract *<br>* page 120, left-hand column, line 9 - line 19 *<br>* page 121, left-hand column, line 3 - line 7 * | 1-9, 11-28 | TECHNICAL FIELDS SEARCHED (IPC)<br>A61K<br>A61M<br>A61P |
| X | DARKE A C ET AL: "Re: Use of rectal medicationsin palliative care",<br>JOURNAL OF PAIN AND SYMPTOM MANAGEM,<br>ELSEVIER, NEW YORK, NY, US,<br>vol. 13, no. 6, 1 June 1997 (1997-06-01),<br>pages 314-315, XP004790311,<br>ISSN: 0885-3924 | 1-28 | |
| Y | * page 315, left-hand column, last full paragraph *<br>* page 315, right-hand column, line 12 *<br>* page 315, right-hand column, line 32 - line 40 * | 1-9, 11-28 | |

-/--

The present search report has been drawn up for all claims

| Place of search | Date of completion of the search | Examiner |
|---|---|---|
| Munich | 30 April 2018 | Terenzi, Carla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 18 15 0895

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | BURDICK MICHAEL ET AL: "Comparison of the Pharmacokinetics of Ketorolac Tromethamine After Continuous Subcutaneous Infusion and Repeat Intramuscular Bolus Injections in Healthy Adult Subjects.", CLINICAL PHARMACOLOGY IN DRUG DEVELOPMENT JUL 2017, vol. 6, no. 4, July 2017 (2017-07), pages 343-349, XP002780601, ISSN: 2160-7648 * the whole document * | 1-28 | |

TECHNICAL FIELDS
SEARCHED        (IPC)

The present search report has been drawn up for all claims

| Place of search | Date of completion of the search | Examiner |
|---|---|---|
| Munich | 30 April 2018 | Terenzi, Carla |

EPO FORM 1503 03.82 (P04C01)

page 2 of 2

## ANNEX TO THE EUROPEAN SEARCH REPORT
## ON EUROPEAN PATENT APPLICATION NO.

EP 18 15 0895

This annex lists the patent family members relating to the patent documents cited in the above-mentioned European search report.
The members are as contained in the European Patent Office EDP file on
The European Patent Office is in no way liable for these particulars which are merely given for the purpose of information.

30-04-2018

| Patent document cited in search report | Publication date | Patent family member(s) | Publication date |
|---|---|---|---|
| WO 2009087658 A2 | 16-07-2009 | NONE | |

EPO FORM P0459

For more details about this annex : see Official Journal of the European Patent Office, No. 12/82

## REFERENCES CITED IN THE DESCRIPTION

**Patent documents cited in the description**

- US 4089969 A
- US 553238 A
- US 6191285 B
- US 6197976 B
- US 6323344 B
- US 8834454 B
- US 20090093772 A
- US 20140148761 A
- US 3910260 A
- US 4004577 A
- US 4689042 A
- US 4755169 A
- US 4795433 A
- US 3941130 A
- US 4261358 A
- US 5085642 A
- US 5092843 A
- US 5102393 A
- US 5267963 A
- US 6149626 A
- US 6270479 B
- US 8679061 B
- US 6371939 B

**Non-patent literature cited in the description**

- **BEAL SL ; SHEINER LB.** *The NONMEM System. Am. Stat.,* 1980, vol. 34, 118
- NONMEM Installation Guide. **BOECKMANN A.J. ; BEAL S.L. ; SHEINER L.B.** NONMEM Users Guide. NONMEM Project Group, University of California, March 1998
- **GLANTZ SA ; SLINKER BA.** Primer of Applied Regression and Analysis of Variance. McGraw Hill, 1990, 225
- **KARLSSON MO.** *Model-building diagnostics, DIA Meeting,* December 2005
- **KARLSSON MO ; SAVIC RM.** Diagnosing model diagnostics. *Clin Pharmacol Ther,* 2007, vol. 82 (1), 17-20
- **YANO Y ; BEAL SL ; SHEINER LB.** Evaluating pharmacokinetic/pharmacodynamic models using the predictive check. *J Pharmacokinet Pharmacodyn,* 2001, vol. 28 (2), 171-92
- **MROSZCZAK EJ ; JUNG D ; YEE J ; COMBS D ; BYNUM L ; SEVELIUS H ; MASSEY I.** Ketorolac tromethamine pharmacokinetics and metabolism after intravenous, intramuscular and oral administration in humans and animals. *Pharmacotherapy,* 1990, vol. 10, 33S-39S
- **TORADOL(R.** Pfizer Labs. 2010
- **TORADOL(R.** *Pfizer Labs,* 2010